(19)

**Europäisches Patentamt**

**European Patent Office**

**Office européen des brevets**

(11) **EP 3 533 520 A2**

(12) **EUROPEAN PATENT APPLICATION**

(43) Date of publication:
**04.09.2019 Bulletin 2019/36**

(51) Int Cl.:
**B01L 3/00** (2006.01)     **B01L 99/00** (2010.01)
**C12Q 1/00** (2006.01)     **A61B 5/145** (2006.01)

(21) Application number: **19152756.3**

(22) Date of filing: **21.01.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.01.2018 JP 2018015740**

(71) Applicant: **SYSMEX CORPORATION**
**Kobe-shi**
**Hyogo 651-0073 (JP)**

(72) Inventors:
• **Kojima, Junko**
 **Hyogo, 651-0073 (JP)**
• **Uchiyama, Kenichi**
 **Hyogo, 651-0073 (JP)**
• **Tokunaga, Kazutoshi**
 **Hyogo, 651-0073 (JP)**
• **Hori, Nobuyasu**
 **Hyogo, 651-0073 (JP)**
• **Morishita, Yu**
 **Hyogo, 651-0073 (JP)**

(74) Representative: **Hoffmann Eitle**
 **Patent- und Rechtsanwälte PartmbB**
 **Arabellastraße 30**
 **81925 München (DE)**

(54) **APPARATUS AND METHOD FOR MEASURING COMPONENTS CONTAINED IN AN INTERSTITIAL FLUID SAMPLE**

(57)     The in-vivo component measuring apparatus 1 for measuring components contained in interstitial fluid collected from a subject is provided. The apparatus 1 includes a setting unit 20 in which is installed an interstitial fluid collector 110 that collects interstitial fluid, a glucose sensor 21 for acquiring a signal reflecting the amount of the measurement target component contained in the interstitial fluid when in a state of contact with the interstitial fluid collector 110, and a moving unit 60 that brings the glucose sensor 21 into contact with the interstitial fluid collector 110 installed in the setting unit 20 by changing the relative position between the setting unit 20 and the glucose sensor 21 to a predetermined positional relationship.

EP 3 533 520 A2

**Description**

FIELD OF THE INVENTION

The present invention relates to an in-vivo component measuring apparatus and an in-vivo component measuring method for measuring in-vivo components contained in interstitial fluid collected from a subject.

BACKGROUND

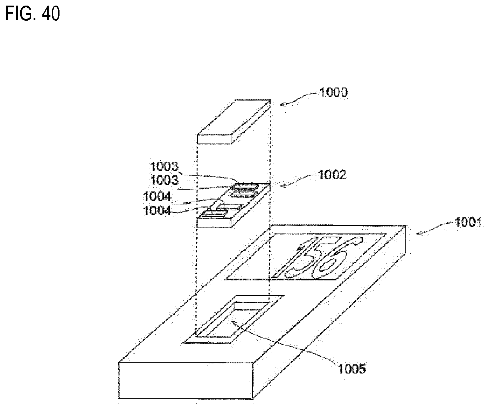

As a method and apparatus for measuring in-vivo components contained in interstitial fluid collected from a subject, a measuring method and a measuring apparatus described in WIPO Publication No. 2010/013808 are known. In WIPO Publication No. 2010/013808, after a process is performed in which micropores are formed on the skin of a subject using a puncture tool to promote exudation of the interstitial fluid from the skin of the subject, then a gel is applied to the skin on which micropores were formed for a predetermined period of time, and the interstitial fluid exudate is collected from the skin in the gel. Then, as shown in FIG. 40, the gel 1000 in which the interstitial fluid has been collected is placed in a setting unit 1005 in a state of contacting electrode units 1003 and 1004 of a sensor chip 1002 of a measuring apparatus 1001, respectively, then the glucose and the sodium ion concentrations are measured, and the area under the blood glucose-time curve (blood glucose AUC) of the subject is calculated based on the measured concentrations of glucose and sodium ions.

SUMMARY OF THE INVENTION

In WIPO Publication No. 2010/013808, the gel 1000 is manually placed on the sensor chip 1002, and the glucose and sodium ion concentrations contained in the interstitial fluid are measured. Therefore, since the positional relationship between the gel 1000 and the electrode units 1003 and 1004 varies from measurement to measurement because the force and angle of pressing the gel 1000 against the sensor chip 1002 varies depending on the user, there is room for improvement inasmuch as the components contained in the gel 100 may not be stably and accurately measured.

A first aspect of the present invention relates to an in-vivo component measuring apparatus 1. The in-vivo component measuring apparatus 1 according to this aspect is an apparatus for measuring a component contained in interstitial fluid collected from a subject and includes a setting unit 20 in which is installed a collector 110 that collects interstitial fluid, an acquiring unit 21 that acquires a signal reflecting the amount of the measurement target component contained in the interstitial fluid when in a state of contact with the collector 110 installed in the setting unit 20, and a moving unit 60 that brings the collector 110 installed in the setting unit 20 into contact with the acquiring unit 21 by changing the relative position between the setting unit 20 and the acquiring unit 21 to a predetermined positional relationship. The "predetermined positional relationship" refers to a positional relationship in which the relative distance, the arrangement direction, and the like of the acquiring unit 21 relative to the setting unit 20 are within a predetermined range, and if the relative position between the setting unit 20 and the acquiring unit 21 is in this positional relationship, the acquiring unit 21 comes into contact with the collector 110 installed in the setting unit 20.

According to the first aspect of the present invention, the acquiring unit 21 is brought into contact with the collector 110 by automatically changing the relative position between the setting unit 20 and the acquiring unit 21 to a predetermined positional relationship. Therefore, it is possible to suppress variations in the positional relationship between the collector 110 and the acquiring unit 21 at the time of measurement from measurement to measurement, as compared with manual placement. Hence, it is possible to stably and precisely measure the collector that has collected the interstitial fluid.

In the first aspect of the present invention, it also is preferable to further include an analyzing unit that generates a value related to the amount of the measurement target component based on a signal reflecting the amount of the measurement target component acquired by the acquiring unit. In this way the value related to the amount of the measurement target component can be generated by the same apparatus.

In the first aspect of the present invention, the acquiring unit 21 preferably is moved to a predetermined position relative to the setting unit 20, thereby changing the relative position between the setting unit 20 and the acquiring unit 21 to a predetermined positional relationship. In this way it is possible to automatically change the relative position between the setting 20 and the acquiring unit 21 to a predetermined positional relationship with a simple operation. "Predetermined position" refers to a position where the relative distance of the acquiring unit 21 relative to the setting unit 20 is within a predetermined range. In this case, the moving unit 60 may move the acquiring unit 21 in the vertical direction relative to the setting unit 20.

It is preferable to further include position detecting sensors 9 and 19 for detecting the position of the acquiring unit 21; the moving unit 60 moves the acquiring unit 21 to a predetermined position based on detection signals from the position detecting sensors 9 and 19. In this way the acquiring unit 21 can be accurately positioned at a predetermined position. More preferably, the position detecting sensor 19 detects a reference position of the acquiring unit 21, and the moving unit 60 moves the acquiring unit 21 a fixed distance from the reference position, thereby moving the acquiring unit 21 to a predetermined position. In this way the acquiring unit 21 can be accurately positioned at a predetermined position with a simple configuration. The "fixed distance" refers to a preset distance, and the numerical value of the distance may have a range.

In the first aspect of the present invention, it is preferable that while the acquiring unit 21 acquires a signal reflecting the amount of the measurement target component, the moving unit 60 maintains a state in which the acquiring unit 21 is pressed against the collector 110 installed in the setting unit 20. In this way the acquiring unit 21 can reliably acquire a signal relating to the amount of the measurement target component contained in the interstitial fluid in the collector 110.

In the first aspect of the present invention, it is preferable that the moving unit 60 includes a pressure adjusting part 61 for adjusting the contact pressure to a constant pressure when the acquiring part 21 contacts the collector 110 to a constant. In this way it is possible to further suppress variations in the contact state between the acquiring unit 21 and the collector 110 since the acquiring unit 21 can be brought into contact with the collector 110 with a constant contact pressure. Therefore, a signal reflecting the amount of the measurement target component contained in the interstitial fluid in the collector 110 can be acquired more stably and accurately. The "constant contact pressure" refers to a preset contact pressure, and the pressure numerical value may have a range.

In this case, the pressure regulator 61 includes a pressure absorbing member 217 provided in the acquiring unit 21. The pressure regulator 61 also includes a pressure absorbing member 2014 provided in the setting unit 20. In this way the acquiring unit 21 can be brought into contact with the collector 110 with a constant contact pressure even if the shape of the collector 110 varies.

The moving unit 60 preferably includes an angle adjuster 216 that adjusts the angle at which the acquiring unit 21 makes contact with the collector 110. In this way the acquiring unit 21 can accurately acquire a signal reflecting the amount of the measurement target component contained in the interstitial fluid in the collector 110 even if the shape of the collector 110 varies since the acquiring unit 21 can be brought into good contact with the collector 110¥

The moving unit 60 preferably moves the setting unit 20 and the acquiring unit 21 such that the movement direction of the setting unit 20 and the movement direction of the acquiring unit 21 intersect, and more preferably moves the setting unit 20 in a horizontal direction and moves the acquiring unit 21 in a vertical direction. In this way it is possible to simplify the configuration for moving the setting unit 20 and the acquiring unit 21.

In the first aspect of the present invention, it is preferable to further include a collector detecting sensor 8 for detecting whether the collector 110 is installed in the setting unit 20. In this way it is possible to confirm whether the collector 110 is installed in the setting unit 20 before measurement of the collector 110.

In the first aspect of the present invention, the setting unit 20 preferably includes a positioning unit 2001 for positioning the collector 110. In this way it is possible to position the collector 110 at an appropriate position relative to the setting unit 20, so that the acquiring unit 21 can be brought into good contact with the collector 110, and the acquiring unit 21 can be placed in the interstitial fluid in the collector 110 to accurately acquire a signal reflecting the amount of the measurement target component contained therein.

In the first aspect of the present invention, a plurality of collector 110 preferably are installed in the setting unit 20, and the moving unit 60 brings the acquiring unit 21 into contact with each of the collectors 110 with respect to the plurality of collectors 110 installed in the setting unit 20. In this way it is possible to reduce the burden on the user when continuously measuring a plurality of collectors 110.

In the first aspect of the present invention, it is preferable to further include a second acquiring unit 22 that acquires a signal reflecting the amount of electrolyte contained in the interstitial fluid when in a state of being in contact with the collector 110, and the moving unit 60 brings the collector 110 installed in the setting unit 20 into contact with the second acquiring unit 22 by changing the relative position between the setting unit 20 and the second acquiring unit 22 to a predetermined positional relationship. It is possible to suppress variations in the positional relationship between the collector 110 and the second acquiring unit 22 at the time of measurement, so that a signal reflecting the amount of the electrolyte contained in the interstitial fluid can be stably and accurately acquired. In this way it is possible to make the analysis result more reliable by using a signal reflecting the amount of the electrolyte to generate a value relating to the amount of the measurement target component more accurately.

The moving unit 60 preferably controls a drive source 23 to move the setting unit 20 between a setting position at which the collector 110 is installed, a measuring position at which the collector 110 and the acquiring unit are in contact, and a second measuring position at which the second acquiring unit 22 and the collector 110 are in contact. In this way the setting unit 20 moves between the different acquiring units 21 and 22, so that the measurement operation can be made more efficient and simplified.

It is preferable that the collector 110 that collects the interstitial fluid and a second collector 111 that collects perspiration from the subject are installed in the setting unit 20, the moving unit 60 brings the second collector 111 installed in the setting unit 20 into contact with the second acquiring unit 22 by changing the relative position between the setting unit 20 and the second acquiring unit 22 to a predetermined positional relationship, and the second acquiring unit 22 also acquires a signal reflecting the amount of the electrolyte contained in the perspiration in the second collector 111. In this way it is possible to make the analysis result highly reliable by reliably and accurately measuring the second collector 111 which collects perspiration and considering the influence of perspiration mixed in the interstitial fluid.

In the first aspect of the present invention, it is preferable to further include a tank 31 for storing the cleaning liquid and a pump 40 for delivering the cleaning liquid from the tank 31, and the moving unit 60 moves the setting unit 20 from the measurement position after measuring the collector 110 by the acquiring unit 21, and the pump 40 sends cleaning liquid to the acquiring unit 21 to perform cleaning. In this way after the measurement of the collector 110, the acquiring unit 21 can be automatically cleaned in preparation for the next measurement.

It is preferable to further include a tank 31 for storing the cleaning liquid and a pump 40 for delivering the cleaning liquid from the tank 31, the moving unit 60 moves the setting unit 20 after the collector 110 is measured by one of the acquiring unit where the collector 110 is measured by the other acquiring unit, and the pump 40 sends cleaning liquid to one acquiring unit to perform cleaning. In this way the cleaning of each acquiring unit can be performed efficiently, and the efficiency of the measurement operation can be improved since the cleaning of one of the acquiring units is performed during the measurement by the other acquiring unit.

A second aspect of the present invention relates to a method for measuring in-vivo components. The in-vivo component measuring method according to this aspect is a method for measuring a component contained in interstitial fluid collected from a subject, and includes a step of horizontally moving the setting unit 20 in which the collector 110 that has collected the interstitial fluid is installed to the measuring position, a step of bringing the component detecting sensor 21 down to a predetermined position relative to the setting unit 20 at the measurement position to bring component detecting unit 21 into contact with the collector 110 installed in the setting unit 20, a step of acquiring a signal reflecting the amount of the measurement target component contained in the interstitial fluid by the component detecting sensor 21 in a state where the component detecting sensor 21 is in contact with the collector 110, a step of generating a value relating to the amount of the measurement target component based on a signal reflecting the amount of the acquired measurement target component.

According to the second aspect of the present invention, the contact between the collector 110 and the component detecting sensor 21 can be easily automated simply by bringing the collector 110 and the component detecting sensor 21 into contact with each other by a simple operation of moving the setting unit 20 horizontally and lowering the component detecting sensor 21 to a predetermined position. Since the component detecting sensor 21 and the collector 110 are brought into contact with each other by lowering the component detecting sensor 21 to a predetermined position, it is possible to suppress variations in the positional relationship between the collector 110 and the component detecting sensor 21 for each measurement by collecting more components than the case where the collector 110 and the component detecting sensor 21 are manually brought into contact. Hence, it is possible to stably and precisely measure the collector that has collected the interstitial fluid.

A third aspect of the present invention relates to a method for measuring in-vivo components. The in-vivo component measuring method according to this aspect is a method for measuring a component contained in interstitial fluid collected from a subject, and includes a step of changing the relative position between the setting unit 20 in which the collector 110 that has collected the interstitial fluid is installed and the component detecting sensor 21 to a predetermined positional relationship, a step of bringing the component detecting sensor 21 into contact with the collector 110 installed in the setting unit 20 with a constant pressure in a predetermined positional relationship, a step of acquiring a signal reflecting the amount of the measurement target component contained in the interstitial fluid by the component detecting sensor 21 in a state where the component detecting sensor 21 is in contact with the collector 110, a step of generating a value relating to the amount of the measurement target component based on a signal reflecting the acquired amount of the measurement target component. In this way it is possible to suppress variations in the positional relationship between the collector 110 and the component detecting sensor 21 for each measurement and to make the acquiring unit 21 contact the collector 110 with a constant contact pressure so as to acquire a signal reflecting the amount of the measurement target component contained in the interstitial fluid in the collector 110 with high accuracy.

In the second and third aspects of the present invention, it is preferable that the component detecting sensor 21 includes a pair of a working electrode and a counter electrode, and includes a pretreatment step of alternately repeating the application of a potential higher and a potential lower than the measurement potential. In this way the detection sensitivity of the measurement target component by the component detecting sensor 21 can be maintained with high sensitivity over a long period.

In the second and third aspects of the present invention, it is preferable that the interstitial fluid is collected from the skin of the subject subjected to a treatment for promoting exudation of the interstitial fluid, and the value related to the amount of the measurement target component is an integrated value of the concentration of the component to be measured in the collecting time of the interstitial fluid. Since this integrated value is obtained by integrating the concentration of the in-vivo measurement target component within the sampling time of the interstitial fluid, it is possible to determine how long the in-vivo measurement target component has been maintained in the high concentration state based on the integrated value.

BRIEF DESCRIPTION OF THE DRAWINGS

FIG. 1 is a flowchart showing a procedure for measuring blood glucose AUC by a method for measuring in-vivo components;

FIG. 2 is a perspective view of a puncture tool;

FIG. 3 is a perspective view of a microneedle chip;

FIG. 4 is an explanatory view of a skin cross section in which micropores are formed by a puncture tool;

FIG. 5A is a plan view of a holding body holding a collector and a second collector, and

FIG. 6 is an explanatory view showing a procedure for collecting interstitial fluid with a collector;

FIG. 7A is a perspective view when the first cover of the in-vivo component measuring apparatus is in a closed state, and FIG. 7B is a perspective view in the opened state;

FIG. 8A is an explanatory view briefly showing an internal structure of an in-vivo component measuring apparatus in a side view, and FIG. 8B is an explanatory view in a front view;

FIG. 9 is a perspective view of a detecting unit.

FIG. 10A is a plan view of the sample plate, FIG. 10B is a side view, and FIG. 10C is a cross-sectional view taken along the line B-B of FIG. 10A;

FIG. 11A is a plan view of a state in which a collector and a second collector are placed on a sample plate, and FIG. 11B is a cross-sectional view taken along line C-C of FIG. 11A;

FIG. 12A is a plan view of the sample stage, FIG. 12B is a cross-sectional view taken along the line D-D of FIG. 12A, and FIG. 12C is a cross sectional view taken along line E-E of FIG. 12A;

FIG. 13A is a perspective view of a glucose sensor and a sodium ion sensor, and FIG. 13B is a side view thereof;

FIG. 14A is a plan view of a fixture and FIG. 14B is a rear view;

FIG. 15 is an explanatory diagram briefly showing a schematic structure of a horizontal movement driving unit in a front view;

FIG. 16 is an explanatory diagram briefly showing a schematic structure of a horizontal movement driving unit in a plan view;

FIG. 17 is an explanatory diagram describing a position where the setting unit moves;

FIG. 18A is an explanatory diagram showing a schematic configuration of a vertical movement driving unit in a plan view, and FIG. 18B is an explanatory diagram shown in a rear view;

FIG. 19 is an explanatory view for explaining a movement range of a glucose sensor and a sodium ion sensor;

FIG. 20 is an explanatory view for explaining the positions where a glucose sensor and a sodium ion sensor move;

FIG. 21 is an explanatory diagram showing a schematic configuration of a sample storage unit and a liquid transfer unit;

FIG. 22 is a block diagram of an in-vivo component measuring apparatus;

FIG. 23 is a flowchart showing a process sequence by a control unit;

FIG. 24 is a flowchart showing details of a calibration process;

FIG. 25A is a flowchart showing details of a measurement process;

FIG. 25B is a flowchart showing details of the measurement process continued from FIG. 25A;

FIG. 25C is a flowchart showing details of the measurement process continued from FIG. 25A;

FIG. 26 is a flowchart showing details of an analysis process;

FIG. 27 is an explanatory diagram illustrating a modified example of the measurement mode of the collector;

FIG. 28 is an explanatory diagram illustrating a modified example of the measurement mode of the collector;

FIG. 29 is a flowchart showing details of a measurement process in a modified example;

FIG. 30 is a flowchart showing details of an analysis process in a modified example;

FIG. 31 is an explanatory view illustrating a modified example of the measurement mode of the collector;

FIG. 32 is an explanatory diagram illustrating a modified example of the measurement mode of the collector;

FIG. 33 is a flowchart showing details of a measurement process in a modified example;

FIG. 34 is a flowchart showing details of an analysis process in a modified example;

FIG. 35 is an explanatory diagram illustrating a modified example of the measurement mode of the collector;

FIG. 36 is an explanatory diagram illustrating a modified example of the measurement mode of the collector;

FIG. 37 is a flowchart showing details of a measurement process in a modified example;

FIG. 38 is a graph showing the detection sensitivity of a glucose sensor;

FIG. 39 is a graph showing time-dependent change in relative detection sensitivity of a glucose sensor; and

FIG. 40 is a perspective view of a conventional in- vivo component measuring apparatus.

DESCRIPTION OF THE EMBODIMENTS OF THE INVENTION

Hereinafter, embodiments of the in-vivo component measuring apparatus and the in-vivo component measuring method of the present invention will be described in detail with reference to the accompanying drawings.

In the present embodiment, an example in which the present invention is applied to the case of measuring the area under the glucose-time curve (hereinafter referred to as "glucose AUC") will be described. Blood glucose AUC is the area (unit: mg · h/dl) of a portion circumscribed by the horizontal axis and a curve drawn by a graph representing the time course of blood glucose level. Blood glucose AUC is an index used for judging the effect of insulin or an oral agent in diabetes treatment. For example, it is possible to estimate the total amount of glucose circulating in the subject's body after glucose loading by measuring the value reflecting the total amount of glucose (blood glucose) circulating in the blood after a glucose load (after meal) within a predetermined period by blood glucose AUC. The total amount of glucose circulating in the subject's body after glucose loading is extremely useful information for knowing how long the hyperglycemic state due to glucose tolerance has lasted. For example, this value becomes a clue to know the secretory response rate of insulin after glucose tolerance, or it becomes a clue to know the effect of diabetes oral medication or insulin when they are administered.

The significance of measuring blood glucose AUC in this manner is that measurement of blood glucose AUC can suppress the influence of individual differences in glucose metabolism in glucose tolerance evaluation by blood glucose measurement at one time point. That is, since there are individual differences in the time until the reaction responds to the blood glucose level due to glucose load, it is not possible to grasp whether the glucose level is at peak by measuring the blood glucose level at a certain point after glucose loading. Even if it is possible to measure the blood glucose level at peak time, it is not possible to grasp how long the hyperglycemic state has lasted. In recent years, diseases of "hidden diabetes mellitus" have attracted attention, but the characteristic of this disease is that although the blood glucose level at the time of fasting is normal or only slightly high, the rise in blood glucose level after meals is steep, and the speed with which the blood glucose value falls afterward is slow and the state of hyperglycemia is longer lasting than that of healthy subjects. Therefore, the blood glucose level measurement at one time point can not clarify how long the hyperglycemic state lasted, and it is obviously not able to provide useful information for screening for hidden diabetes. In this regard, if blood glucose AUC is measured, a value reflecting the total amount of blood glucose circulated in the blood within a predetermined period can be obtained, so that the measured value is influenced by the time until the response appears in the blood glucose level due to glucose loading, and how long the hyperglycemic state has lasted can be estimated based on the measured value. In this way, it is possible to obtain a value useful for estimating the glucose tolerance due to glucose tolerance without being influenced by individual differences in glucose metabolism by measuring glucose AUC.

Usually, blood glucose AUC is measured by collecting blood at predetermined time intervals (for example, at intervals of 30 minutes) and acquiring the blood glucose levels of the collected blood, respectively. Then, a blood glucose value AUC is obtained by acquiring a graph showing the time course of the blood glucose level and obtaining the area of the part surrounded by the curve drawn by the graph and the horizontal axis. The value of blood glucose AUC obtained by using the measuring apparatus and the measuring method according to the following embodiments can be used for judging diabetes instead of the measured value of blood glucose AUC by such blood sampling.

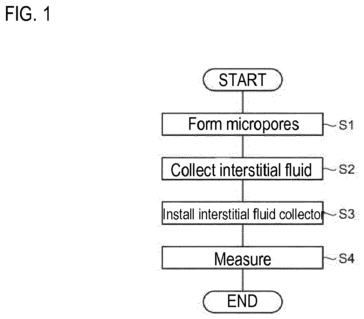

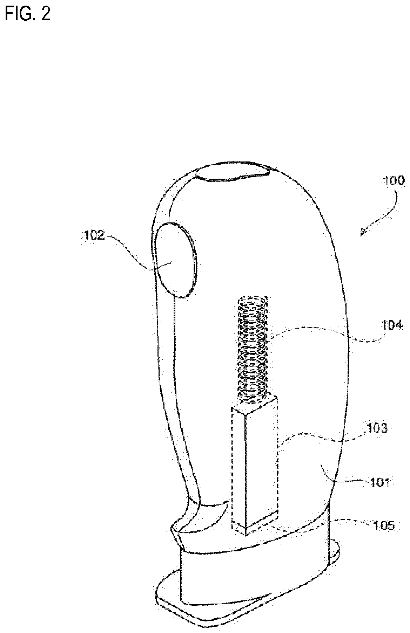

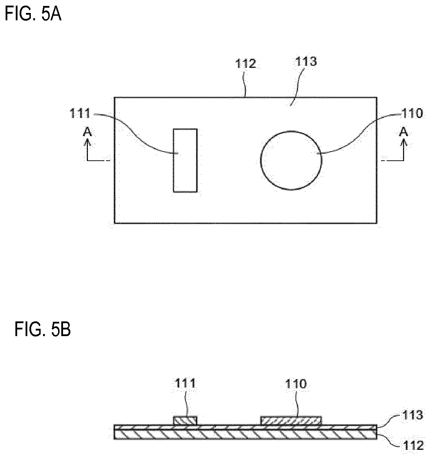

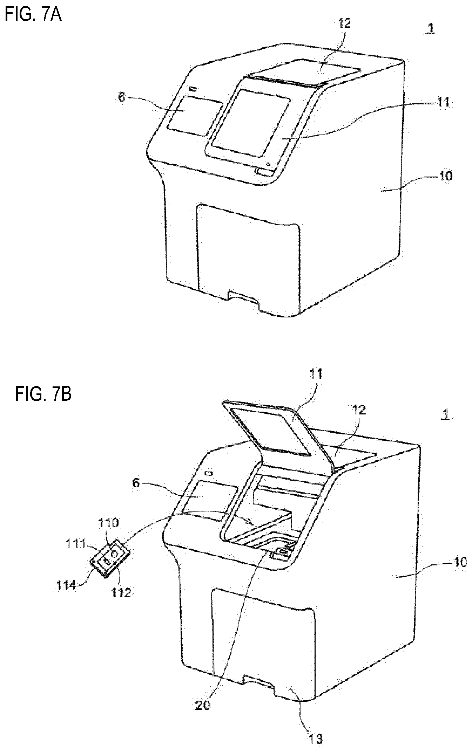

In the measurement of blood glucose AUC according to the in-vivo component measuring method of the present embodiment, a plurality of micropores first are formed on the skin of a subject using a puncture tool 100 (shown in FIG. 2) as a treatment to promote exudation of the interstitial fluid, and the interstitial fluid is exuded through the plurality of micropores as shown in step S1 of FIG. 1, the details of which will be described later. Next, in step S2 the interstitial fluid exuded using the collector 110 (shown in FIG. 5; hereinafter referred to as "interstitial fluid collector 110") is collected, glucose as a measurement target component containing interstitial fluid accumulates in the interstitial fluid collector 110, and electrolyte (sodium ions) accumulate as an auxiliary component. Next, in step S3, the interstitial fluid collector 110 is set in the setting unit 20 (shown in FIG. 7) of the in-vivo component measuring apparatus 1. Next, in step S4, a signal relating to the concentration of glucose and a signal relating to the concentration of sodium ions are obtained as a signal reflecting the amount of glucose and a signal reflecting the amount of electrolyte of the measurement target component accumulated in the interstitial fluid collector 110, and measured using the in-vivo component measuring apparatus 1 to determine glucose concentration and sodium ion concentration. Then, a blood glucose AUC is calculated based on the glucose concentration and the sodium ion concentration as values relating to the amount of the measurement target component.

When the subject perspires, sodium ions derived from perspiration are accumulated in the collector from the skin of the subject so as to be superposed on the sodium ions derived from the interstitial fluid, and the sodium ion concentration is increased. In the in-vivo component measuring method of the present embodiment, since the blood glucose AUC of the subject is measured based on sodium ions accumulated together with glucose, the measured blood glucose AUC may be reduced in some cases if excessive accumulation of sodium ions derived from perspiration occurs. Therefore, in the in-vivo component measuring method of the present embodiment, in step S2 the interstitial fluid collector 110 for main measurement is fixed to the region where the micropores are formed in the subject's skin, and a second collector 111 (shown in FIG. 5; hereinafter referred to as "perspiration collector 111") for perspiration check is fixed in a region where no micropores are formed, such that perspiration from the skin is collected using the perspiration collector 111 to accumulate sodium ions contained in the perspiration accumulated in the perspiration collector 111 at the same time as collecting the interstitial fluid exuding from the skin using the interstitial fluid collector 110. Then, in S4, the sodium ion concentration derived from perspiration accumulated in the perspiration collector 111 is measured, and the blood glucose AUC is calculated with high reliability by considering the sodium ion concentration derived from perspiration at the time of calculating the blood glucose AUC.

Configurations of the in-vivo component measuring apparatus 1, the puncture tool 100, the interstitial fluid collector 110, and the perspiration collector 111 used in the in-vivo component measuring method of the present embodiment will be described hereinafter.

Puncture Tool

FIG. 2 shows an example of the puncture tool 100. The puncture tool 100 is a device that forms many micropores in the skin of a subject, for example, the skin of an arm, to promote exudation of interstitial fluid from the skin of the subject. The puncture tool 1 00 includes a housing 101, a release button 102 provided on the surface of the housing 101, an array chuck 103 provided inside a housing 100, a spring member 104 for a downward force on the array chuck 103. An opening (not shown) is formed in a lower portion of the housing 100. A micro needle chip 105 subjected to sterilization treatment can be attached to the lower part of the array chuck 103.

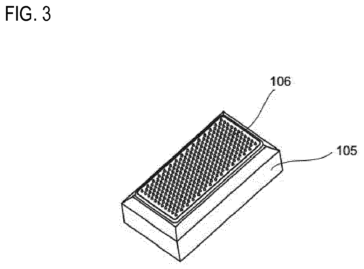

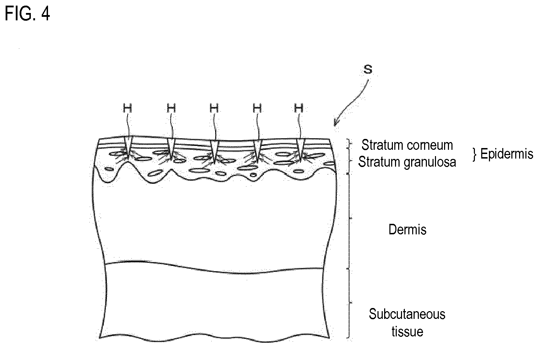

As shown in FIG. 3, the microneedle chip 105 is provided with a plurality of fine needles 106 on its lower surface. As shown in FIG. 4, the puncture tool 100 punctures the epidermis (the skin of the subject) of the living body with the plurality of microneedles 106, whereby the micropores H are formed for exuding the interstitial liquid on the skin S of the subject. The plurality of microneedles 106 has such a depth that the plurality of micropores H formed remain in the epidermis of the skin S and do not reach the dermis.

The puncture tool 100 also includes a fixing mechanism (not shown) that fixes the array chuck 103 in a state of pushing the array chuck 103 upward against the biasing force of the spring member 104 such that fixation of the array chuck 103 by the fixing mechanism is released when the user such as the subject presses the release button 102. In this way the array chuck 103 moves toward the skin by the force exerted by the spring member 104, and the plurality of fine needles 106 of the microneedle chip 105 protrude from the lower opening of the housing 100 and puncture the skin.

Interstitial Fluid Collector and Perspiration Collector

FIG. 5 shows an example of the interstitial fluid collector 110 and the perspiration collector 111. The interstitial fluid collector 110 is fixed to the skin of the subject in order to collect the interstitial fluid from the skin of the subject, and is removed from the skin after a predetermined time has elapsed. The perspiration collector 111 is fixed to the subject's skin to collect perspiration from the subject's skin, and is removed from the skin after a predetermined time has elapsed, preferably after the same time as the interstitial fluid collector 110. Note that it is preferable that the perspiration collector 111 is fixed in a region close to the region where the interstitial fluid collector 110 is fixed on the subject's skin.

The interstitial fluid collector 110 and the perspiration collector 111 are made of, for example, a gel having water retentivity and capable of holding interstitial fluid and perspiration. The gel is not particularly limited insofar as it is capable of collecting interstitial fluid and perspiration, but a gel formed from at least one hydrophilic polymer selected from the group consisting of polyvinyl alcohol and polyvinyl pyrrolidone is preferable. The hydrophilic polymer forming the gel may be polyvinyl alcohol alone or polyvinyl pyrrolidone alone, or may be a mixture of both, but it is preferred that the gel is polyvinyl alcohol alone or a mixture of polyvinyl alcohol and polyvinyl pyrrolidone.

The gel can be formed by crosslinking the hydrophilic polymer in an aqueous solution. The gel can be formed by a method in which an aqueous solution of a hydrophilic polymer is applied onto a substrate to form a coating film, and the hydrophilic polymer contained in the coating film is crosslinked. As a crosslinking method of the hydrophilic polymer, a chemical crosslinking method, a radiation crosslinking method and the like are available, but it is preferable to adopt a radiation crosslinking method from the viewpoint that it is difficult for various chemical substances to be mixed in the gel as impurities.

Although the interstitial fluid collector 110 has a circular shape in a plan view and the perspiration collector 111 has a rectangular shape in plan view in this embodiment, the shape of the interstitial fluid collector 110 and the perspiration collector 111 is not limited to this. The size (volume) of the interstitial fluid collector 110 and the perspiration collector 111 is determined according to the collection time of interstitial fluid and perspiration.

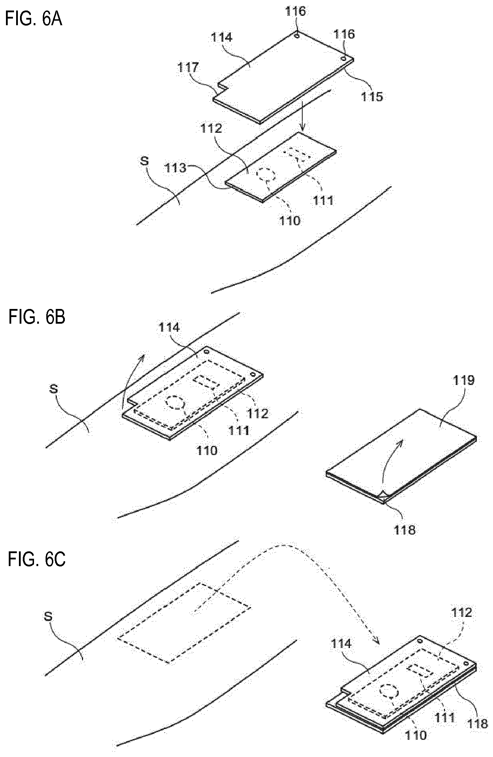

In the present embodiment, the interstitial fluid collector 110 and the perspiration collector 111 are held by a holding sheet 112 having a rectangular shape in plan view. The holding sheet 112 is flexible and transparent, and is made of a resin material such as polyethylene terephthalate. A transparent pressure-sensitive adhesive layer 113 is formed on one side of the holding sheet 112, and the interstitial fluid collector 110 and the perspiration collector 111 are attached to the pressure-sensitive adhesive layer 113 at intervals in the longitudinal direction. As shown in FIG. 6A, the interstitial fluid collector 110 and the perspiration collector 111 are arranged such that the interstitial fluid collector 110 covers a region where a plurality of micropores are formed in the skin S of the subject, and the adhesive layer 113 of the sheet 112 is affixed to the skin S of the subject. At this time, the perspiration collector 111 is arranged in a region where the plurality of micropores are not formed in the skin S of the subject. Note that prior to use the interstitial fluid collector 110 and the perspiration collector 111 are sealed by the holding sheet 112 and a release film (not shown) is affixed to one surface of the holding sheet 112 via the adhesive layer 113.

When removing the interstitial fluid collector 110 and the perspiration collector 111 from the subject's skin S, a support sheet 114 shown in FIG. 6A can be used. The support sheet 114 is rectangular in plan view and has a contour that is slightly larger than the holding sheet 112. The support sheet 114 is flexible and transparent, and is made of a resin material such as polyethylene terephthalate.

A transparent adhesive layer 115 is formed on one side of the support sheet 114, and the support sheet 114 is adhered by the adhesive layer 115 on the other side of the holding sheet 112 (the side on which the interstitial fluid collector 110 and the perspiration collector 111 are not adhered). The force of the pressure-sensitive adhesive layer 115 of the support sheet 114 is stronger than the adhesive force of the pressure-sensitive adhesive layer 113 of the holding sheet 112, thereby supporting the pressure-sensitive adhesive layer 115 of the support sheet 114 in a state of being adhered to the holding sheet 112, and by separating the sheet 114 from the skin S the holding sheet 112 can be smoothly removed together with the interstitial fluid collector 110 and the perspiration collector 111 from the skin S.

In the present embodiment, the interstitial fluid collector 110 and the perspiration collector 111 are subjected to measurement while being supported by the support sheet 114. In order to accurately measure the interstitial fluid collector 110 and the perspiration collector 111, the support sheet 114 is formed with small diameter through holes 116 at two corner portions on one end side in the longitudinal direction. A notch 117 having a rectangular shape in a plan view also is formed at one corner portion on the other end side in the longitudinal direction of the support sheet 114.

The interstitial fluid collector 110 and the perspiration collector 111 removed from the subject's skin S can be protected by a protective sheet 118 shown in FIG. 6B. The protective sheet 118 is rectangular in plan view and has a contour approximately the same size as the support sheet 114. The protective sheet 118 is flexible and transparent, and is made of a resin material such as polyethylene terephthalate. Prior to use of the holding sheet 118, a release film 119 is adhered on one surface. The release film 119 is for protecting one side of the protective sheet 118 from contamination. In order to easily peel off the release film 119 from the protective sheet 118, the application surface of the release film 119 is a weakly viscous adhesive surface. Note that a release treatment such as silicone coating may be applied to one surface of the protective sheet 118 so that the release film 119 can be easily peeled off.

As shown in FIG. 6C, the release film 119 is peeled off from the protective sheet 118, and one side of the protective sheet 118 is overlaid on the surface of the support sheet 114 on the side where the interstitial fluid collector 110 and the perspiration collector 111 are adhered, the protective sheet 118 is adhered by the adhesive layer 115 of the support sheet 114. In this way the interstitial fluid collector 110 and the perspiration collector 111 supported by the support sheet 114 are sealed by the protective sheet 118.

After the interstitial fluid and the perspiration are collected from the skin of the subject with the interstitial fluid collector 110 and the perspiration collector 111, the interstitial fluid collector 110 and the perspiration collector 111 can be stored without being contaminated immediately when the interstitial fluid collector 110 and the perspiration collector 111 are not subjected to measurement by sealing the interstitial fluid collector 110 and the perspiration collector 111 with the support sheet 114 and the protective sheet 118 in this way. The interstitial fluid collector 110 and the perspiration collector 111 also can be transported to the measurement site without being contaminated when the interstitial fluid or perspiration collection location and the measurement location are separated.

Note that the support sheet 114 and the protective sheet 118 are not necessarily separate sheets. The support sheet 114 and the protective sheet 118 may be integrated so that the support sheet 114 can be folded over the protective sheet 118.

In-vivo Component Measuring Apparatus

The in-vivo component measuring apparatus 1 includes a setting unit 20 where an interstitial fluid collector 110 that collects interstitial fluid is installed, a glucose sensor 21 that acquires a signal reflecting the amount of the measurement target component contained in the interstitial fluid in contact with the interstitial fluid collector 110 installed in the setting unit 20, and a moving unit 60 that brings the glucose sensor 21 into contact with the interstitial fluid collector 110 installed in the setting part 20 by changing the relative position between the setting part 20 and the glucose sensor 21 to a predetermined positional relationship. The in-vivo component measuring apparatus 1 acquires a signal reflecting the amount of glucose contained in the interstitial fluid by the glucose sensor 21, and measures the glucose concentration in the interstitial fluid. In the present embodiment, the in-vivo component measuring apparatus 1 includes a sodium ion sensor 22 that acquires a signal reflecting the amount of the electrolyte contained in the interstitial fluid while being in contact with the interstitial fluid collector 110 installed in the setting unit 20, and the moving unit 60 changes the relative position between the setting unit 20 and the sodium ion sensor 22 to a predetermined positional relationship so as to produce contact of the interstitial fluid collector 110 installed in the setting unit 20 and the sodium ion sensor 22,and acquire a signal reflecting the amount of sodium ions contained in the interstitial fluid, and the sodium ion concentration in the interstitial fluid is measured. Then, based on the measured glucose concentration and sodium ion concentration, the in-vivo component measuring apparatus 1 calculates the blood glucose AUC of the subject and generates and displays an analysis result including blood glucose AUC.

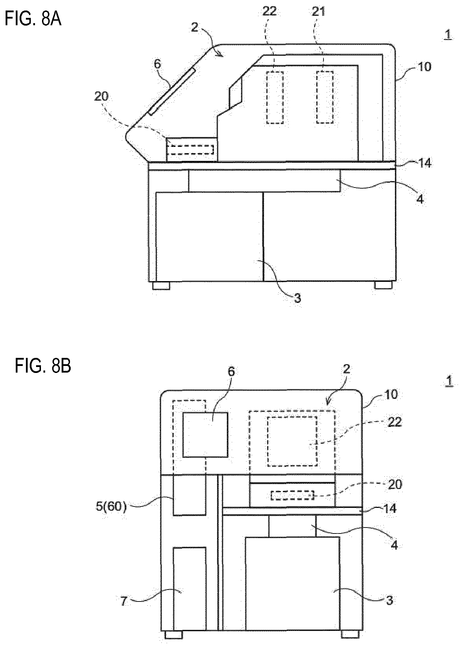

As shown in FIGS. 7 and 8, the in-vivo component measuring apparatus 1 includes a detecting unit 2, a reagent storage unit 3, a liquid transfer unit 4, a control unit 5, an operation display unit 6, and a power supply 7, and these units are provided in a housing 10.

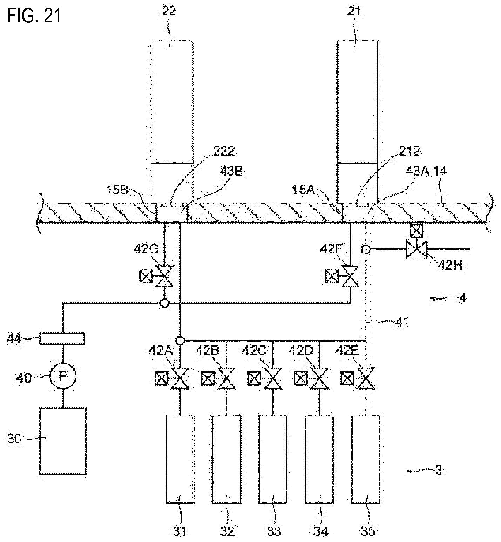

A first cover 11 is provided at a position adjacent to the operation display unit 6 on the upper front of the housing 10. The first cover 11 is a push-open type cover, and the first cover 11 is erected by pushing, and the first cover 11 is brought into the open state shown in FIG. 7B from the closed state shown in FIG. 7A to expose the setting unit 20 for the installation of the interstitial fluid collector 110. A second cover 12 is provided on the upper surface of the housing 10. The second cover 12 is also a push-open type cover, and although not shown in the drawing, the second cover 12 is erected by pushing to be changed from the closed state to the open state to expose the glucose sensor 21 and the sodium ion sensor 22. A third cover 13 is provided at the front lower portion of the housing 10. When the third cover 13 is opened, the various tanks 30 to 35 (shown in FIG. 21) of the reagent storage 3 are exposed.

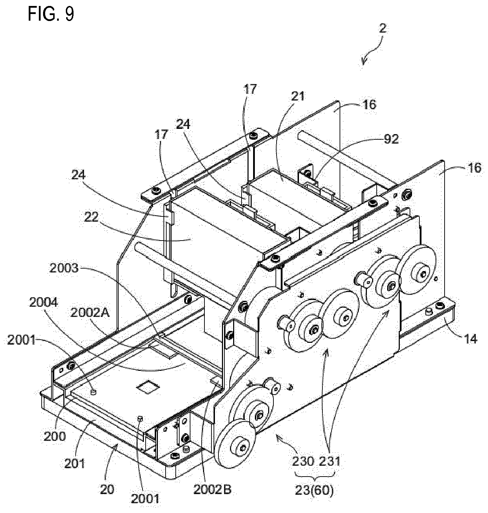

The detecting unit 2 acquires a signal related to the amount of component (glucose or electrolyte) contained in the interstitial fluid collected in the interstitial fluid collector 110, and a signal on the amount of component (electrolyte) contained in the perspiration collected in the perspiration collector 111. As shown in FIG. 9, the detecting unit 2 includes a setting unit 20, a glucose sensor 21, a sodium ion sensor 22, and a drive unit 23.

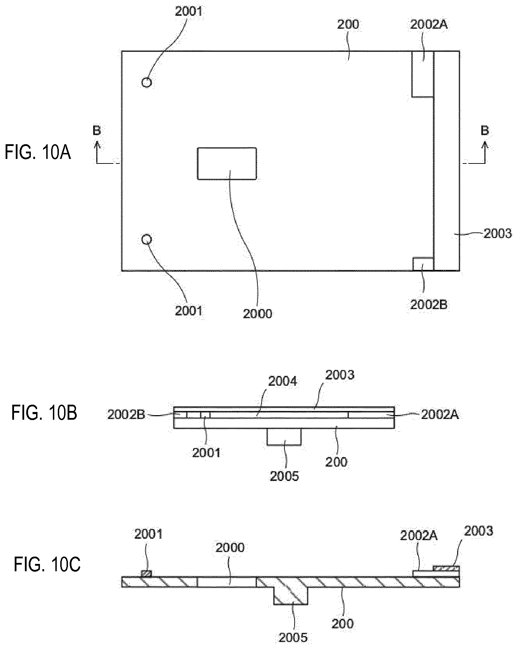

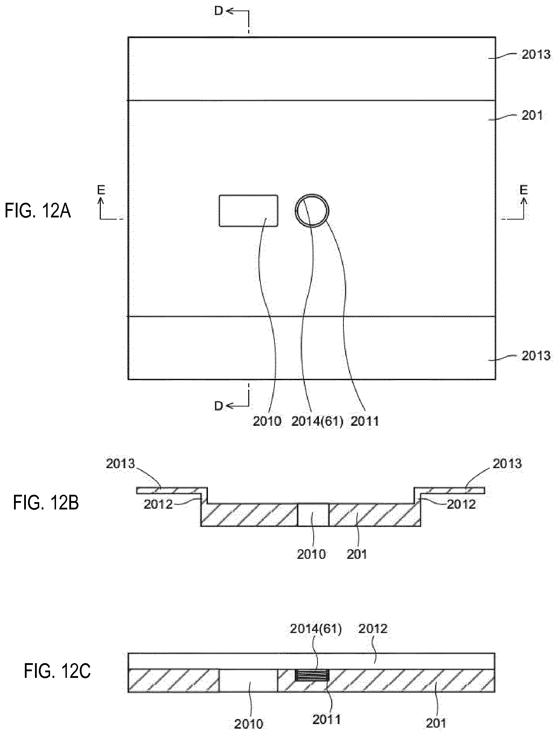

An interstitial fluid collector 110 and a perspiration collector 111 are installed in the setting unit 20. The setting unit 20 is configured by a sample plate 200 (see FIG. 10) on which a support sheet 114 supporting the interstitial fluid collector 110 and the perspiration collector 111 is placed, and a sample stage 201 on which the sample plate 200 is installed (shown in FIG. 12).

As shown in FIG. 10, the sample plate 200 has a rectangular shape in plan view and has a contour that is slightly larger than the support sheet 114. The upper surface of the sample plate 200 is a flat surface, and the support sheet 114 can be placed stably on the sample plate 200. In this way the sensors 21 and 22 can be brought into good contact with the interstitial fluid collector 110 and the perspiration collector 111 when measuring the interstitial fluid collector 110 and the perspiration collector 111.

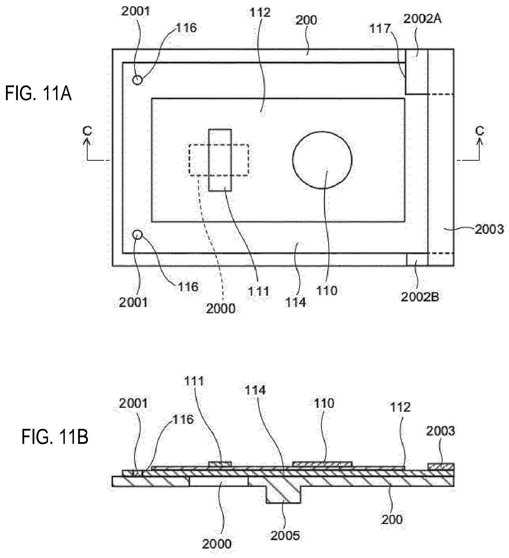

Small protrusions 2001 are respectively provided at two corner portions on one end side in the longitudinal direction of the sample plate 200. The two small protrusions 2001 function as positioning parts for positioning the interstitial fluid collector 110 and are formed on the support sheet 114, and are fitted into the two through holes 116 when the support sheet 114 is placed on the sample plate 200 as shown in FIG. 11. In this way the support sheet 114 is placed on the sample plate 200 without being displaced, so that the interstitial fluid collector 110 and the like can be positioned at appropriate positions with respect to the sample plate 200. As shown in FIG. 10, vertical walls 2002A and 2002B having the same thickness or slightly greater as the support sheet 114 are provided at two corner portions on the other end side in the longitudinal direction of the upper surface of the sample plate 200. As shown in FIG. 11, when the support sheet 114 is placed on the sample plate 200, one vertical wall 2002A strikes the notch 117 of the support sheet 114 and the other vertical wall 2002B is along the side edge opposite to the notch 117 of the support sheet 114. In this way the support sheet 114 can be positioned more effectively at an appropriate position on the sample plate 200. By holding the support sheet 114 on the sample plate 200 without misalignment in this way, the sensors 21 and 22 can be brought into good contact with each other when measuring the interstitial fluid collector 110 and the perspiration collector 111.

As shown in FIG. 10, a horizontal bar 2003 is bridged between the two vertical walls 2002A and 2002B on the other end side in the longitudinal direction of the sample plate 200, and an insertion hole 2004 is formed between the upper surface of the sample plate 200 and the horizontal bar 2003. As shown in FIG. 11, when the support sheet 114 is placed on the sample plate 200, a part of the support sheet 114 on the other end side in the longitudinal direction is inserted in the insertion hole 2004. As a result, the support sheet 114 is prevented from floating on the sample plate 200 by the horizontal bar 2003, and can be stably placed on the sample plate 200. In this way the sensors 21 and 22 can be brought into good contact with the interstitial fluid collector 110 and the perspiration collector 111 when measuring the interstitial fluid collector 110 and the perspiration collector 111.

As shown in FIG. 10, a first detection hole 2000 also is formed in the sample plate 200 at a position where the interstitial fluid collector 110 or the perspiration collector 111 is placed. An engaging convex part 2005 also is provided at the center of the lower surface of the sample plate 200.

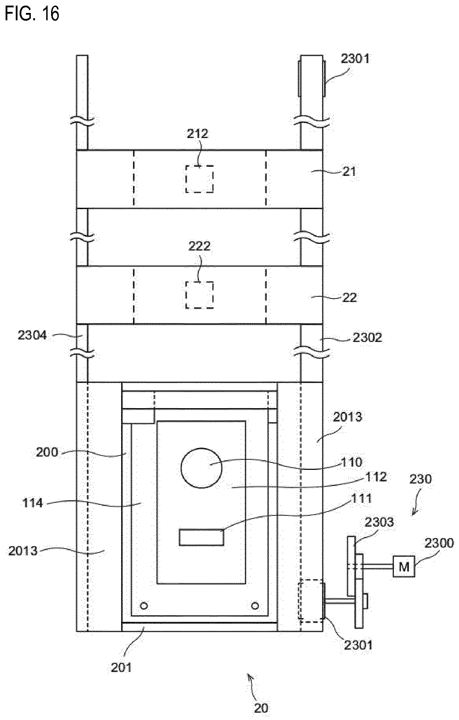

As shown in FIG. 12, the sample stage 201 has a rectangular shape in plan view, and has a contour that is slightly larger than the sample plate 200. The sample plate 200 is installed on the upper surface of the sample stage 201. On both side edges of the upper surface of the sample stage 201, side walls 2012 extending in the longitudinal direction are erected, and protrusions 2013 protrude outward in the horizontal direction from the upper end portion of each side wall 2012. The sample stage 201 is reciprocated in the X direction along the horizontal plane as shown in FIG. 16 by the horizontal movement drive unit 230 of the drive unit 23 described later. In this way the interstitial fluid collector 110 and the perspiration collector 111 are conveyed to positions below the respective sensors 21 and 22.

An engaging concave part 2011 is formed at the center of the upper surface of the sample stage 201. When the sample plate 200 is placed on the sample stage 201, the engaging convex part 2005 of the sample plate 200 fits into the engaging concave part 2011. A pressure absorbing member 2014 is accommodated in the engaging concave part 2011, and the engaging convex part 2005 is supported by the pressure absorbing member 2014 in the engaging concave part 2011. A spring member such as a coil spring can be used as the pressure absorbing member 2014, for example. The pressure absorbing member 2014 configures a pressure regulator 61 for regulate a constant contact pressure when each sensor 21 and 22 makes contact with the interstitial fluid collector 110, and is included in the moving unit 60. When the sensors 21 and 22 come into contact with the interstitial fluid collector 110 and the perspiration collector 111, the pressure absorbing member 2014 expands and contracts, and the sample plate 200 is displaced upward and downward on the sample stage 201, so that it is possible to adjust the contact pressure of the electrode units 212 and 222 in contact with the perspiration collector 111 to be a constant pressure. In the present embodiment, specifically, it is possible to adjust the contact pressure to IN (tolerance ± 2 to 3%). Therefore, even if there are variations in the shapes of the interstitial fluid collector 110 and the perspiration collector 111, the electrode units 212 and 222 can be brought into contact with the interstitial fluid collector 110 and the perspiration collector 111 at a constant contact pressure. Note that a single pressure absorbing member 2014 interposed between the sample plate 200 and the sample stage 201 is not provided at the center of the sample plate 200 and the sample stage 201, for example, one pressure absorbing member 2014 is provided for each of the four corners of the sample plate 200 and the sample stage 201 four total), and there are no particular restrictions on the installation position.

A second detection hole 2010 is formed in the sample stage 201. The second detection hole 2010 is formed at a position coinciding with the first detection hole 2000 of the sample plate 200 when the sample plate 200 is placed on the sample stage 201. The first detection hole 2000 and the second detection hole 2010 configure a detecting unit for confirming whether the interstitial fluid collector 110 and the like are installed in the sample stage 201.

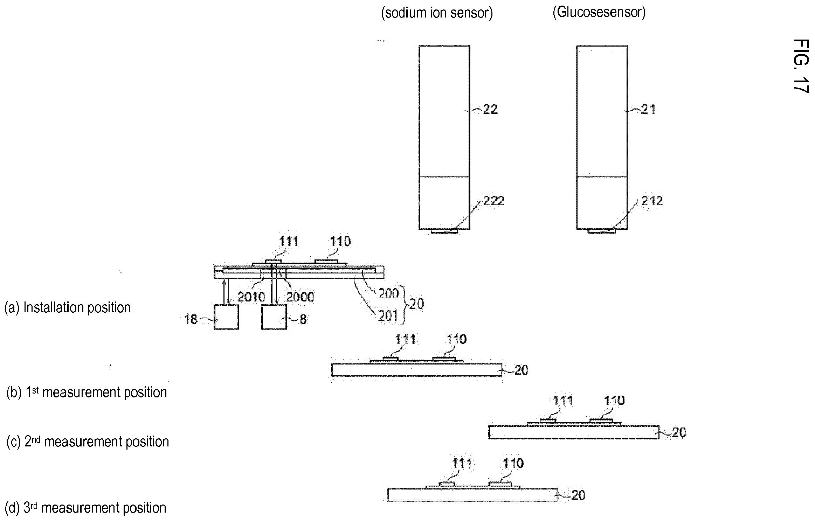

The in-vivo component measuring apparatus 1 includes a collector detecting sensor 8 (shown in (a) of FIG. 17) such as a photosensor in the housing 10. The collector detecting sensor 8 configures the detecting unit and emits light upward below the sample stage 201 at an installation position at which the sample plate 200 on which the interstitial fluid collector 110 and the like are set is placed on the sample stage 201.

When the interstitial fluid collector 110 and the like is not installed on the sample stage 201, the light emitted from the light emitting element of the collector detecting sensor 8 does not enter the light receiving element, whereas the light emitted from the light emitting element of the collector detecting sensor 8 is reflected by the interstitial fluid collector 110 or the perspiration collector 111 and is incident on the light receiving element when the interstitial fluid collector 110 is installed on the sample stage 201. The collector detecting sensor 8 is connected to a control unit 5, and when the light receiving element receives light, an electric signal is output to the control unit 5. The control unit 5 detects that the interstitial fluid collector 110 and the like are installed on the sample stage 201 based on the electric signal from the collector detecting sensor 8.

Note that although in the present embodiment, the collector detecting sensor 8 is a reflection type photosensor (photoreflector), a transmission type photosensor (photointerrupter) also may be used. The collector detecting sensor 8 is not limited to the photosensor, and also may be any object detecting sensor that can detect the installation of the interstitial fluid collector 110 and the like on the sample stage 201 in a noncontact manner.

The glucose sensor 21 is a component detecting sensor that acquires a signal reflecting the amount of glucose which is a measurement target component contained in the interstitial fluid, and functions as an acquiring unit. The sodium ion sensor 22 also is a component detecting sensor that acquires a signal reflecting the amount of sodium ions as an auxiliary component contained in the interstitial fluid, and functions as a second acquiring unit.

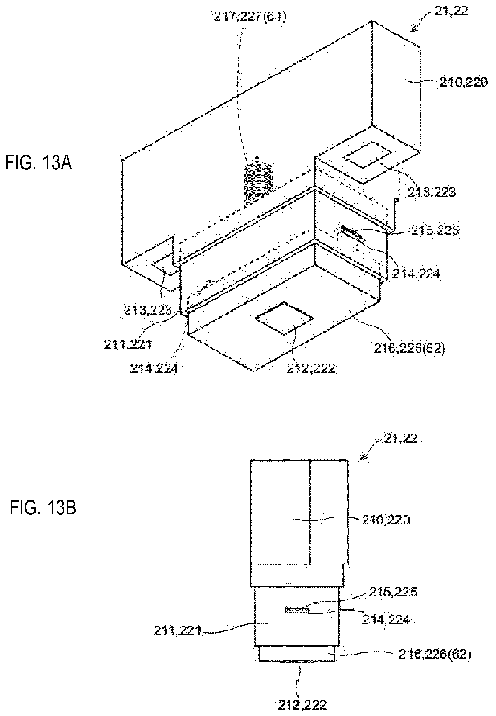

As shown in FIG. 13, each of the sensors 21 and 22 includes, for example, plastic body 210 and 220, slide parts 211 and 221 that are slidably attached to the body 210 and 220, plastic cartridge 216 and 226 that are detachably attached to the cartridge 211 and 221, and electrode units 212 and 222 that are attached to the lower surface of the cartridges 216 and 226.

The body 210, 220 has an upper part and a lower part and has a shape having a step between the upper part and the lower part, and terminals 213, 223 connected to the control unit 5 are provided on the lower surface of the upper part. Openings are formed in the lower portions of the body 210 and 220, and the slide 211 and 221 protrude from the openings. Pressure absorbing members 217 and 227 are provided in the body 210 and 220. For the pressure absorbing members 217 and 227, for example, a spring member such as a coil spring can be used. The pressure absorbing members 217 and 227 configure a pressure regulator 61 for regulating a constant contact pressure when each sensor 21 and 22 makes contact with the interstitial fluid collector 110, and are included in the moving unit 60. The slides 211, 221 are connected to the pressure absorbing members 217, 227 and slide up and down with respect to the main body 210, 220 by expansion and contraction of the pressure absorbing members 217, 227. When the sensors 21 and 22 come into contact with the interstitial fluid collector 110 and the perspiration collector 111, the pressure absorbing members 217 and 227 expand and contract, and the electrode units 212 and 222 are displaced upward and downward, whereby it is possible to regulate a constant contact pressure of the electrode units 212 and 222 in contact with the interstitial fluid collector 110 and perspiration collector 111. Therefore, even if there are variations in the shapes of the interstitial fluid collector 110 and the perspiration collector 111, the electrode units 212 and 222 can be brought into contact with the interstitial fluid collector 110 or the perspiration collector 111 at a predetermined contact pressure. Note that a plurality of pressure absorbing members 217, 227 connected to the slides 211, 221 in the main body 210, 220 may be provided instead of one.

An opening is formed in the lower portion of the slides 211, 221, and the cartridges 216, 226 can be attached to the lower portion of the slides 211, 221 through this opening. Engaging holes 215 and 225 are formed on both side surfaces of the lower portions of the slides 211, 221.

The cartridges 216, 226 are expendable items that are disposable when they are subjected to the measurement of the interstitial fluid collector 110 or the like for a predetermined number of times. The cartridges 216, 226 are provided with a pair of engaging claws 214, 224 so as to correspond to the engaging holes 215, 225 of the slides 211, 221. The engaging claws 214 and 224 are engaged with the corresponding engaging holes 215 and 225, respectively, so that the cartridges 216 and 226 are held by the slides 211 and 221. At this time, it is preferable that the cartridges 216, 226 are held stationary relative to the slides 211, 221, for example, so as to be able to oscillate by being slightly tapped. The cartridges 216, 226 configure an angle adjuster 62 for adjusting the angle at which the sensors 21, 22 come into contact with the interstitial fluid collector 110, and are included in the moving unit 60. In this way when the sensors 21 and 22 contact the interstitial fluid collector 110 and the perspiration collector 111, the cartridges 216, 226 oscillate along the surfaces of the interstitial fluid collector 110 and the perspiration collector 111 relative to the slides 211, 221. Therefore, it is possible to adjust the angle of the surface of the electrode units 212, 222 in contact with the interstitial fluid collector 110 and the perspiration collector 111, and the electrode units 212, 222 can be brought into good contact with the interstitial fluid collector 110 and the perspiration collector 111 even if the interstitial fluid collector 110 or the perspiration collector 111 varies in shape.

The electrode units 212 and 222 include a pair of a working electrode and a counter electrode, and a reference electrode. For example, the glucose sensor electrode unit 21 for glucose measurement of the glucose sensor 21 has a working electrode configured by a platinum electrode unit and a glucose oxidase enzyme membrane formed thereon, and the counter electrode is configured by a platinum electrode unit. On the other hand, the electrode unit 222 for sodium ion measurement of the sodium ion sensor 22 has, for example, a working electrode configured by an ion selective electrode unit having a sodium ion selective membrane, and a counter electrode configured by a reference electrode.

The glucose sensor 21 includes a circuit for glucose measurement (not shown) as an electric circuit connected to the electrode unit 212, and the glucose sensor 21 applies a constant voltage to the interstitial fluid collected in the interstitial fluid collector 110 via the electrode unit 212 in contact with the interstitial fluid collector 110, and the current at that time is acquired as a detection value. This current value depends on the glucose concentration in the interstitial fluid. On the other hand, the sodium ion sensor 22 includes a sodium ion measurement circuit (not shown) as an electric circuit connected to the electrode unit 222, and the electrode unit 222 comes in contact with the interstitial fluid collector 110 or the perspiration collector 111, whereby the voltage of the interstitial fluid collected in the interstitial fluid collector 110 or the perspiration collected in the perspiration collector 111 is acquired as the detection value. This voltage value depends on the sodium ion concentration in interstitial fluid and perspiration. The glucose sensor 21 and the sodium ion sensor 22 are connected to the control unit 5 and output the obtained current value and voltage value as a detection signal to the control unit 5. The control unit 5 measures the glucose concentration and the sodium ion concentration based on the current value and the voltage value included in the detection signal and a calibration curve stored in the storage unit.

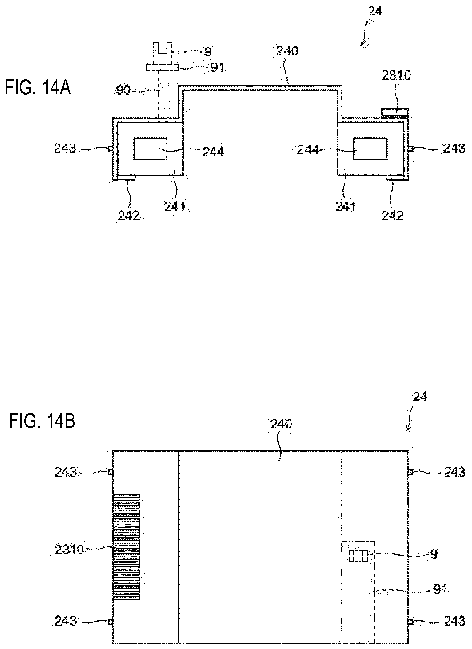

The glucose sensor 21 and the sodium ion sensor 22 are set in the detecting unit 2 by mounting on a fixture 24. As shown in FIG. 14, the fixture 24 includes a frame 240 capable of housing the upper portions of the sensors 21 and 22, a pair of left and right supports 241 for supporting the lower portions of the sensors 21 and 22 from below, and a holding unit 242 for clamping the upper portions of the frames 21 and 22 forward and backward with the frame 240. An opening 244 for exposing the terminals 213 and 223 of the sensors 21 and 22 is formed in each support 241. The fixture 24 is mounted so as to be vertically movable on a pair of left/right side plates 16 (see FIG. 9) provided on the base plate 14 of the detection unit 2. A plurality of protrusions 243 are provided on the both side surfaces of the frame 240 in the vertical direction, and the fixture 24 moves vertically in a straight line by sliding the elongated hole-shaped guide hole 17 (shown in FIG. 9) formed in each side plate 16 in the vertical direction.

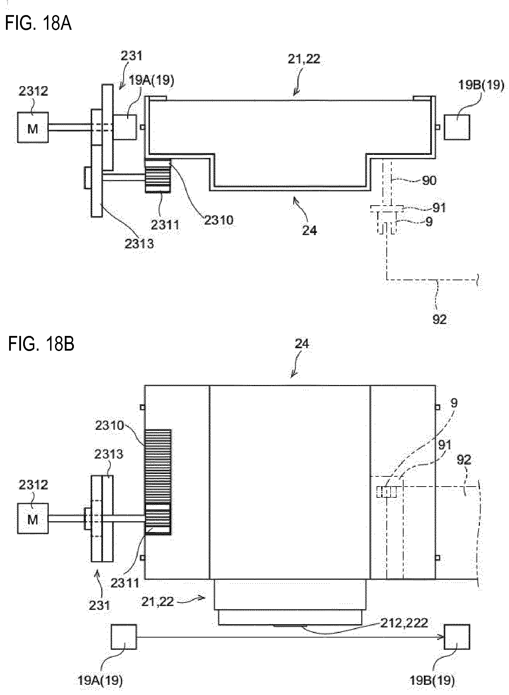

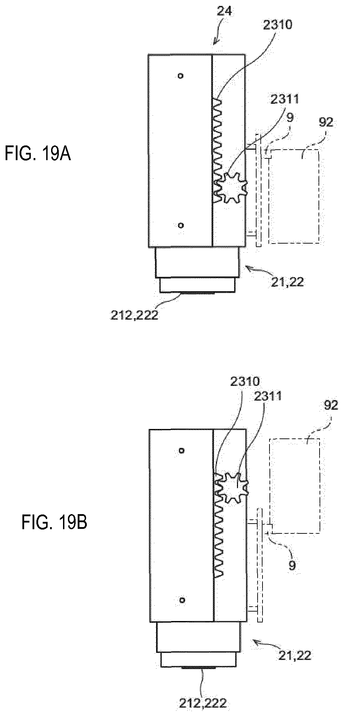

A rack 2310 is provided along the vertical direction on the side portion on the left and right sides of the back surface of the frame 240. The rack 2310 configures a vertical movement drive unit 231 of the drive unit 23 described later, and pinion gears 2311 (shown in FIGS. 18 and 19) to be described later mesh with tooth surfaces of the surface of the rack 2310.

Next, in order to bring the interstitial fluid collector 110 installed in the setting unit 20 into contact with the sensors 21 and 22 in the present embodiment, the drive unit 23 moves the setting unit 20 and the sensors 21 and 22, and is included in the moving unit 60. The drive unit 23 includes a horizontal movement drive unit 230 that moves the setting unit 20 in the horizontal direction and a vertical movement drive unit 231 that moves the glucose sensor 21 and the sodium ion sensor 22 in the vertical direction. The vertical movement drive unit 231 is provided in the detecting unit 2 corresponding to each of the glucose sensor 21 and the sodium ion sensor 22.

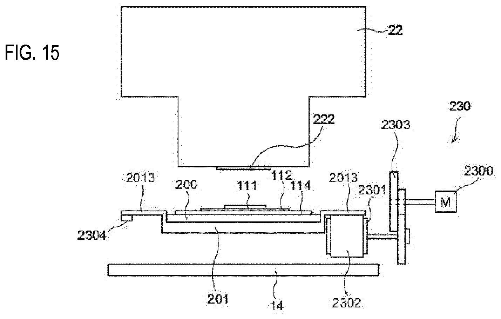

As shown in FIGS. 15 and 16, the horizontal movement drive unit 230 includes a motor 2300 serving as a drive source, a pair of pulleys 2301, a transmission belt 2302 wound around the pair of pulleys 2301, a rotation driving of the motor 2300, a power transmission mechanism 2303 for shifting the rotational drive force of the motor 2300 and transmitting this force to one of the pulleys 2301. The setting unit 20 is fixed to the transmission belt 2302 by one protrusion 2013 of the sample stage 201. The motor 2300 is, for example, a stepping motor that is rotatable in forward and reverse directions, and the horizontal movement drive unit 230 causes the transmission belt 2302 to travel only by a distance corresponding to the rotation angle (rotation number) of the motor 2300, and reciprocates the setting part 20 fixed on the upper part in the X direction along the horizontal plane. Note that the other protrusion 2301 of the sample stage 201 of the setting unit 20 slides on the rail 2304 provided on one side plate 16 (shown in FIG. 9) of the detecting unit 2. The motor 2300 is connected to the control unit 5, and its operation is controlled by the control unit 5. Note that the stepping motor rotates an angle corresponding to the number of drive pulses to be supplied.

The in-vivo component measuring apparatus 1 includes an origin detecting sensor 18 (shown in (a) of FIG. 17) such as a photosensor in the housing 10. In this embodiment, the origin detecting sensor 18 is a reflection type photosensor (photoreflector), which is arranged so as to emit light upward below the sample stage 201 at the installation position. When the setting unit 20 is located at the installation position, the light emitted from the light emitting element of the origin detecting sensor 18 is reflected by the sample stage 201 and is incident on the light receiving element. On the other hand, when the setting unit 20 is not located at the installation position, the light emitted from the light emitting element of the origin detecting sensor 18 does not enter the light receiving element. The origin detecting sensor 18 is connected to the control unit 5, and outputs an electric signal to the control unit 5 when the light receiving element receives the light. The control unit 5 detects that the installation unit 20 is located at the installation position based on the electric signal from the origin detecting sensor 18. The control unit 5 performs the process of setting the installation position where the setting unit 20 is detected by the origin detecting sensor 18 as the origin in the horizontal direction.

Note that although the origin detecting sensor 18 is a reflection type photosensor (photoreflector) in this embodiment, the sensor may be a transmission type photosensor (photointerrupter). The origin detecting sensor 18 is not limited to a photosensor, and may be any object detecting sensor that can detect that the setting unit 20 is located at the installation position in a non-contact manner.

As shown in FIG. 17, the control unit 5 controls the horizontal movement drive unit 230 to move the setting unit 20 from the installation position (shown in (a) of FIG. 17) where the interstitial fluid collector 110 can be installed to each predetermined position. Specifically, the setting unit 20 is moved from the installation position to each position where the distance corresponding to the number of pulses set from the installation position is far away, that is, the first measurement position (see (b) of FIG. 17) where the interstitial fluid collector 110 is located below the sodium ion sensor 22, the second measurement position (shown in (c) of FIG 17) where the interstitial fluid collector 110 is located below the glucose sensor 21, the third measurement position (shown in (d) of FIG. 17) where the perspiration collector 111 is located below the sodium ion sensor 22, by supplying drive signals of the number of pulses respectively set to the motor 2300. In this way the sensors 21 and 22 can come into contact with the interstitial fluid collector 110 and the perspiration collector 111, and the interstitial fluid collector 110 and the perspiration collector 111 are measured.

In this way the setting unit 20 is transported between the installation position, the first measurement position, the second measurement position, and the third measurement position by the horizontal movement drive unit 230. Although the horizontal movement drive unit 230 converts the forward/reverse rotation of the motor 2312 into a reciprocating linear movement by the transmission belt 2302, and transmits the same to the setting unit 20, thereby moving the setting unit 20 in the horizontal direction in the present embodiment, the drive unit 230 also be configured using a power transmission mechanism other than the transmission belt 2302, such as a configuration in which the sample stage 201 is pushed from behind.

In addition, each measurement position at which the setting unit 20 is transported from the installation position is positioned by the control unit 5 which sends the number of drive pulses corresponding to the distance from the installation position to the motor 2300, but a rotary encoder may be used to further improve the positioning accuracy.

As a method for positioning the setting unit 20 at each measurement position, an object detecting sensor such as a photosensor is provided at each measurement position, and the object detecting sensors at each measurement position detect the setting unit 20, so that the setting unit 20 may be detected when reaching the respective measurement positions.

As shown in FIGS. 18 and 19, the vertical movement drive unit 231 includes a rack 2310 provided in the fixture 24, a pinion gear 2311 engaging with the rack 2310, a motor 2312 serving as a drive source, a rotational drive force of the motor 2312, and a power transmission mechanism 2313 for shifting the rotational drive force of the motor 2312 and transmitting the force to the pinion gear 2311. The motor 2312 is, for example, a stepping motor that can rotate in forward and reverse directions, and the vertical movement drive unit 231 vertically moves the fixture 24 by a distance corresponding to the rotation angle (rotation number) of the motor 2312. The motor 2312 is connected to the control unit 5, and the operation of the control unit 5 is controlled by the control unit 5. Note that the stepping motor rotates an angle corresponding to the number of drive pulses to be supplied.

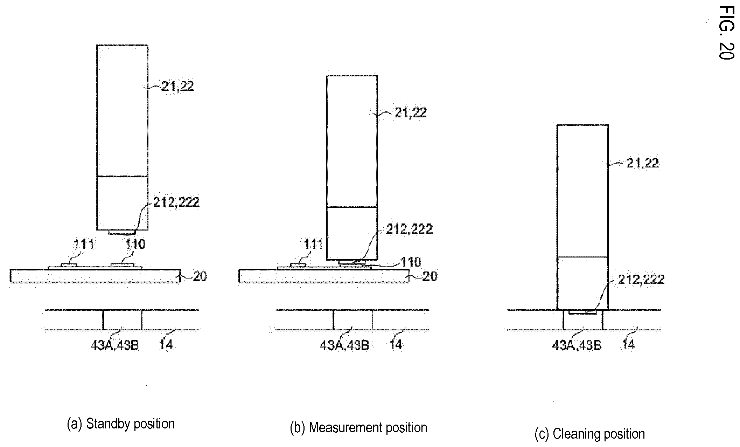

The in-vivo component measuring apparatus 1 includes a top dead center detecting sensor 19 (shown in FIG. 18), such as a photosensor, for example, on one side plate 16 (shown in FIG. 9). In the present embodiment, the top dead center detecting sensor 19 is a transmissive photosensor (photo interrupter), and includes a pair of a light emitter 19A and a light receiver 19B, and the light emitter 19A and the light receiver 19B are connected to the sensors 21 and 22. When the sensors 21 and 22 are positioned at the highest position, the light emitted from the light emitting element of the light emitter 19A of the top dead center detecting sensor 19 is incident on the light receiving element of the light receiver 19B. As shown in FIG. 20A, this uppermost position is a position where each sensor 21, 22 does not participate in the measurement of the interstitial fluid collector 110 or the perspiration collector 111 and the does not participate in cleaning of the electrode units 212, 222, and is a standby position for waiting until there is an instruction from the user. On the other hand, when the sensors 21 and 22 are positioned below the uppermost position (standby position), the light emitted from the light emitting element of the light emitter 19A of the top dead center detecting sensor 19 is transmitted to the sensors 21 and 22 and does not enter the light receiving element of the light receiver 19B. The top dead center detecting sensor 19 is connected to the control unit 5, and when the light receiver 19B receives light, an electric signal is output to the control unit 5. Based on the electric signal from the top dead center detecting sensor 19, the control unit 5 detects that the sensors 21, 22 are positioned at the uppermost position (standby position). The control unit 5 also performs a process for setting the uppermost position (standby position) where each sensor 21, 22 is detected by the top dead center detecting sensor 19 to the uppermost dead point (top dead point) in the vertical direction.

Although the top dead center detecting sensor 19 is a transmissive photosensor (photo interrupter) in the present embodiment, it also may be a reflective photosensor (photo reflector). The top dead center detecting sensor 19 is not limited to a photosensor, and also may be any object detecting sensor that can detect that each sensor 21, 22 is located at the standby position in a non-contact manner.

As shown in FIG. 20, the control unit 5 controls the vertical movement drive unit 231 to move the sensors 21, 22 from the standby position (shown in FIG. 20A) to predetermined positions. Specifically, by supplying the drive signals of the number of pulses respectively set to the motor 2312, it is possible to move from the standby position to each measurement position separated by a distance corresponding to the number of pulses respectively set from the standby position, that is, the measurement position (see FIG. 20B) where the sensors 21 and 22 are in contact with contact the interstitial fluid collector 110 or the perspiration collector 111, and a cleaning position (shown in FIG. 20C) where cleaning of the electrode units 212 and 222, which will be described later, is performed relative to the sensors 21 and 22.

In this way the sensors 21, 22 are transported between the standby position, the measurement position, and the cleaning position by the vertical movement drive unit 231. Note that although the vertical movement drive unit 231 converts forward and reverse rotation of the motor 2312 into reciprocating rectilinear movement by the rack 2310 and the pinion gear 2311 and transmits the drive to the respective sensors 21 and 22, a power transmission mechanism other than the rack 2310 and the pinion gear 2311 also may be used.

The positions to which the above-described sensors 21 and 22 are transported from the standby position, are determined by the control unit 5 giving the number of drive pulses corresponding to the distance from the standby position to the motor 2312, rotary encoders also may be used to further enhance the positioning accuracy.

A position detecting sensor 9 attached to the frame member 240 of the fixture 24 may be used as a method of positioning each sensor 21, 22 at each position, as indicated by two-dot chain lines in FIGS. 14, 18 and 19. The position detecting sensor 9 is fixed to a sensor support plate 91 attached to the frame 240 via a fixture 90, and moves together with the fixture 24 in the vertical direction. In this example, a detection plate 92 (shown in FIGS. 18 and 19) is fixed to one side plate 16 (shown in FIG. 9) of the detecting unit 2 so as to face the position detecting sensor 9. By detecting the upper end and the lower end of the detection plate 92, the position detecting sensor 9 detects the highest position (standby position) and the lowest position cleaning position) of the sensors 21, 22 that move in the vertical direction by the vertical movement drive unit 231. The position detecting sensor 9 is connected to the control unit 5 and outputs an electric signal to the control unit 5 when detecting the upper end and the lower end of the detection plate 92. The position detecting sensor 9 preferably can use a proximity sensor using magnetism or the like so as to detect the detection plate 92, but is not limited to this, inasmuch as various sensors such as an optical sensor can be used. When detecting that the sensors 21, 22 have reached the uppermost position (standby position) and the lowermost position (cleaning position) based on the electric signal from the position detecting sensor 9, the control unit 5 causes the vertical movement drive unit 231 to stop each sensor 21, 22 at each position. Note that positioning of the measurement position may be performed by the control unit 5 giving the motor 2312 a number of drive pulses corresponding to the distance from the uppermost standby position, so that each sensor 21, 22 may detect contact with the collector 110 or the perspiration collector 111. For example, contact of each sensor 21, 22 with the interstitial fluid collector 110 and perspiration collector 111 can be detected by the conduction between the electrode units of the sensors 21, 22.

Next, as shown in FIG. 21, the reagent storage 3 is provided with a waste liquid tank 30, a first tank 31 for storing the cleaning liquid, a second tank 32 for storing a low concentration calibration solution for glucose, a third tank 33 for storing the high concentration calibration solution for glucose, a fourth tank 34 for storing medium concentration calibration solution for sodium ion, and a fifth tank 35 for storing high concentration calibration solution for sodium ion. The cleaning liquid in the first tank 31 is used for cleaning the glucose sensor 21 and the sodium ion sensor 22 and is used for preparing a calibration curve for sodium ions as a low concentration calibration solution for sodium ions. A PB-K solution is an example of a cleaning liquid. Calibration solutions for glucose in the second tank 32 and the third tank 33 are used for preparing a calibration curve of glucose. As a calibration solution for glucose, a PB-K solution to which glucose is added can be exemplified. The glucose concentration of the PB-K solution is, for example, 0.5 mg/dL at a low concentration, and a concentration determined within a range of, for example, 10 mg/dL to 40 mg/dL at a high concentration. The calibration solutions for sodium ions in the fourth tank 34 and the fifth tank 35 are used for preparing a calibration curve of sodium ions. As a calibration solution for sodium ion, saline can be exemplified. The sodium ion concentration of the saline solution is a concentration determined within a range of, for example, 1 mM to 2 mM at a medium concentration, and a concentration determined within a range of, for example, 20 mM to 50 mM at a high concentration. Other examples of the calibration solution for sodium include Tris solution and PB-K solution. The glucose concentration or the sodium ion concentration of each liquid of the tanks 31 to 35 is stored in the storage unit of the control unit 5.

Next, as shown in FIG. 21, the liquid transfer unit 4 supplies the liquid stored in each of the tanks 31 to 35 to the glucose sensor 21 and the sodium ion sensor 22 arranged in the detecting unit 2, and collects the liquid in the waste liquid tank 30. The liquid transfer unit 4 includes a pump 40, a pipe 41 made of a pipe or the like, a plurality of electromagnetic valves 42A to 42H, a first nozzle 43A, and a second nozzle 43B. The nozzles 43A and 43B are fixed to the base plate 14 so as to communicate with the supply holes 15A and 15B formed in the base plate 14. The pump 40 is disposed in a flow path from the waste liquid tank 30 to the respective nozzles 43A, 42B of the pipe 41. The solenoid valves 42A to 42H have a function of switching the opening and closing of the flow path in which each of the pipes 41 is disposed. The pump 40 and the solenoid valves 42A to 42H are connected to the control unit 5, and operation is controlled by the control unit 5.

The first tank 31 is connected to the first nozzle 43 and the second nozzle 44 via the solenoid valve 42A, and supplies cleaning liquid to the glucose sensor 21 and the sodium ion sensor 22 via the nozzles 43A and 43B. The second tank 32 and the third tank 33 are connected to the first nozzle 43A via the solenoid valves 42B and 42C respectively, and the calibration liquid for the first acquiring unit is supplied to the glucose sensor 21 via the first nozzle 43A. The fourth tank 34 and the fifth tank 35 are connected to the second nozzle 43B via the solenoid valves 42D and 42E respectively, and the calibration liquid for the second acquiring unit is supplied to the sodium ion sensor 22 through the second nozzle 43B . The waste liquid tank 35 is connected to the first nozzle 43A via the solenoid valve 42F and to the second nozzle 43B via the solenoid 42G, and is supplied to the glucose sensor 21 and the sodium ion sensor 22 so that the discharged liquid is recovered through the nozzle 43A and 43B after passing through a filter 44.

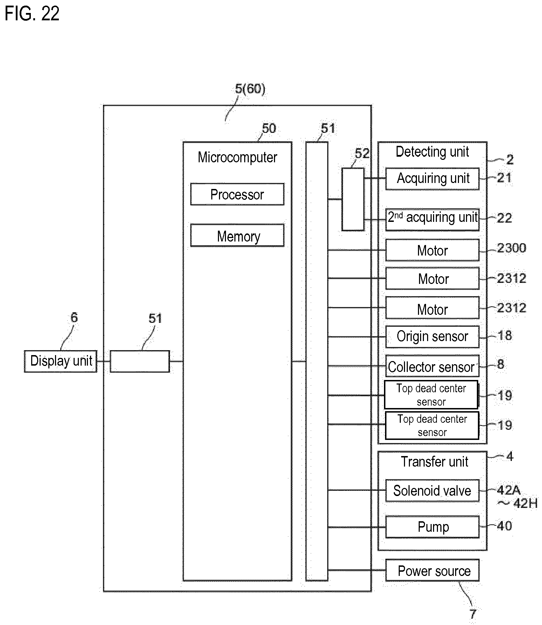

Next, the control unit 5 controls the drive unit 23 so as to bring the interstitial fluid collector 110 installed in the setting unit 20 into contact with the sensors 21, 22, and the structure is included in the moving unit 60. As shown in FIG. 22, the control unit 5 includes a microcomputer 50 which has a processor (for example, CPU) and a memory (for example, ROM and RAM), a user interface control board 51, an I/O board 52, and an analog board 53, and other circuits for processing various signals. The control unit 5 reads and executes a program stored in the ROM by the CPU, thereby controlling the operation of each unit such as the detecting unit 2, the liquid transfer unit 4, the operation display unit 6, and the like. The RAM is used as a development area of the program when the program stored in the ROM is executed. The control unit 5 has the functions of storage unit, control unit for controlling the drive unit 23 of the detecting unit 2, and the analysis unit for calculating the glucose AUC based on signals reflecting the amount of measurement target component (glucose) and amount of electrolyte (sodium ion as an adjunct component mreceived from each sensor 1 and 22 of the detecting unit 2, and the like.

Next, the operation display unit 6 is used for issuing instruction to start the measurement and display analysis results and the like. The operation display unit 6 can be configured by a touch panel type display. Note that the operation display unit 6 may be divided into an operation section and a display section, in which case the operation section can be configured by buttons, switches, a keyboard, and a mouse.

Next, the power supply 7 converts the AC power supply voltage input from the power supply plug (not shown) into a DC voltage and supplies it to the control unit 5. The power supply 7 is also connected to the other parts and supplies electric power to each part.

In-vivo Component Measuring Method

Next, the in-vivo component measurement of the present embodiment will be described. FIG. 1 and FIGS. 23 to 26 are flowcharts of the in-vivo component measuring method of the present embodiment.

First, in step S1 shown in FIG. 1, after cleansing the skin of a subject with alcohol or the like, a process is performed to form micropores in the skin of the subject using the puncture tool 100, and promote exudation of the interstitial fluid from the skin. Next, in step S2, as shown in FIG. 6A, the interstitial fluid exuded from the skin S is collected using the interstitial fluid collector 110 by attaching the holding sheet 112 to the skin S of the subject so that the interstitial fluid collector 110 is arranged in the region where the micropores are formed. At the same time, the perspiration collector 111 for perspiration check is used to collect the perspiration coming out of the skin S of the subject. The sampling time of the interstitial fluid is, for example, about 60 minutes to 180 minutes. Next, in step S3, as shown in FIG. 6B, using the support sheet 114, the holding sheet 112 together with the interstitial fluid collector 110 and the perspiration collector 111 is detached from the skin S of the subject, and the interstitial fluid collector 110 and the perspiration collector 111 removed from the skin S are set on the setting unit 20 of the in-vivo component measuring apparatus 1 while being supported by the supporting sheet 114. Specifically, the support sheet 114 is placed on the sample plate 200, and this sample plate 200 is set on the sample stage 201. When the interstitial fluid collector 110 and the perspiration collector 111 are installed in the setting section 20 of the in-vivo component measuring apparatus 1, next, measurement of the interstitial fluid collector 110 and the perspiration collector 111 is performed by the in-vivo component measuring apparatus 1 by instructing measurement start using the operation display unit 6 in step S4.

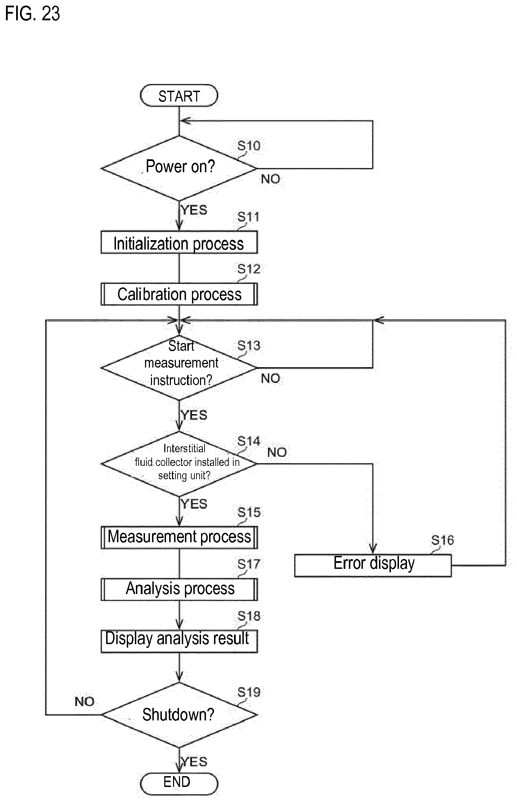

In the in-vivo component measuring apparatus 1, as shown in FIG. 23, when the power of the apparatus is turned on in step S10, the control unit 5 performs an initialization process in step S 11. For example, the setting unit 20 is positioned at the origin in the horizontal direction when the setting unit 20 is positioned at the installation position (shown in (a) of FIG. 17) based on the detection result of the origin detecting sensor 18, and each sensor 21, 22 is in the standby position (shown in FIG. 20A)) when positioned at the top dead center in the vertical direction based on the detection results of the respective position detecting sensors 9.

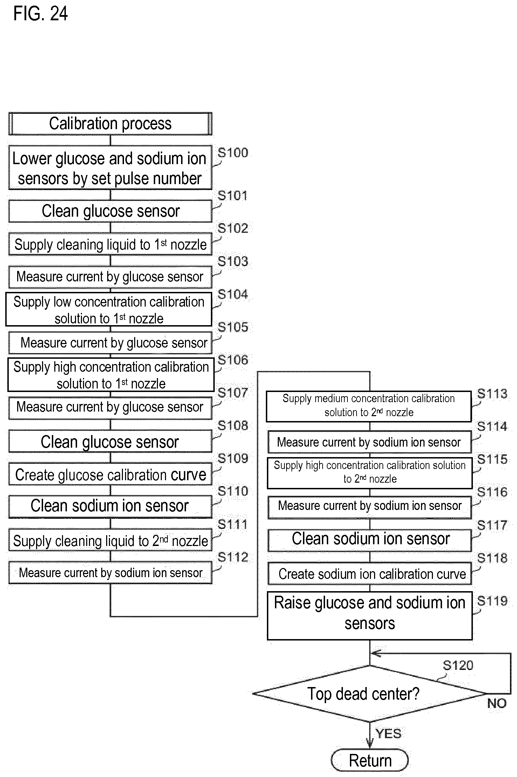

Next, in step S12, the control unit calibrates each sensor 21, 22. FIG. 24 is a flowchart of the calibration process of step S12. In the present embodiment, "calibration" is based on a measurement value obtained by measuring a component contained in a known concentration sample with the glucose sensor 21 or the sodium ion sensor 22 to create a relational expression between measured values and component concentrations acquired by the glucose sensor 21 and the sodium ion sensor 22.

First, in step S100, the control unit 5 controls the motor 2312 of the vertical movement drive unit 231 to lower the glucose sensor 21 and the sodium ion sensor 22 by the set number of pulses from the standby position to the cleaning position (as shown in FIG. 20C).

Next, the control unit 5 cleans the glucose sensor 21 in S101. In this process, the flow path from the first tank 31 through the first nozzle 43A to the pump 40 is opened by opening the solenoid valve 42A, solenoid valve 42F, and solenoid valve 42H and closing the other solenoid valves. In this state, the control unit 5 drives the pump 40 to supply cleaning liquid from the first tank 31 to the first nozzle 43A to clean the electrode units 212 of the glucose sensor 21. The cleaning liquid supplied to the first nozzle 43A is collected in the waste liquid tank 30.

Next, in step S102, the control unit 5 maintains the cleaning liquid supplied to the first nozzle 43A by closing the solenoid valve 42F. Then, in step S103, a constant voltage (for example, 0.45 V) is applied to the electrode units 212 of the glucose sensor 21 while the cleaning liquid is in contact with the electrode units 212 of the glucose sensor 21, and after standing for a predetermined time, the glucose sensor 21 measures the current value I0. The current value I0 at this time is the current value when the glucose concentration is 0 mg/dl. Then, the control unit 5 opens the solenoid valve 42F and closes the solenoid valve 42A, thereby sending the cleaning liquid supplied to the first nozzle 43A to the waste liquid tank 30.

Next, in step S104, the control unit 5 opens the solenoid valve 42B, the solenoid valve 42F and the solenoid valve 42H and closes the other solenoid valves, to open the flow path from the second tank 32 through the first nozzle 43A to the pump 40. In this state, a low-concentration calibration solution for glucose is supplied from the second tank 32 to the first nozzle 43A by driving the pump 40, and thereafter the solenoid valve 42F is closed to maintain low concentration calibration solution for glucose to the first nozzle 43A. Then, in step S105, a constant voltage (for example, 0.45 V) is applied to the electrode unit 212 of the glucose sensor 21 in a state where the low concentration calibration solution for glucose is in contact with the electrode unit 212, and the control unit 5 causes the glucose sensor 21 to measure the current value IL. Then, the control unit 5 opens the solenoid valve 42F and closes the solenoid valve 42B, thereby sending the low-concentration calibration solution for glucose supplied to the first nozzle 43A to the waste liquid tank 30.

Next, in step S106, the control unit 5 opens the solenoid valve 42C, the solenoid valve 42F and the solenoid valve 42H and closes the other solenoid valves, to open the flow path from the third tank 33 through the first nozzle 43A to the pump 40. In this state, a high-concentration calibration solution for glucose is supplied from the third tank 33 to the first nozzle 43A by driving the pump 40, and thereafter the solenoid valve 42F is closed to maintain the supply of high concentration solution for glucose to the first nozzle 43A. Then, in step S107, with a high concentration calibration solution for glucose being in contact with the electrode unit 212 of the glucose sensor 21, the control unit 5 applies a constant voltage (for example, 0.45 V) and causes the glucose sensor 21 to measure the current value IH. Then, the control unit 5 opens the solenoid valve 42F and closes the solenoid valve 42C, thereby delivering the high-concentration calibration solution for glucose supplied to the first nozzle 43A to the waste liquid tank 30.

Next, in step S108, the control unit 5 cleans the glucose sensor 21 in the same manner as in step S101.

Next, in step S109, based on the concentrations of the low concentration calibration solution for glucose and the high concentration calibration solution for glucose stored in the storage unit, a calibration curve of glucose is prepared and stored in the storage unit based on the measured current value IL and current value IH.

Next, the control unit 5 performs the cleaning of the sodium ion sensor 22 in step S110. In this process, the solenoid valve 42A, the solenoid valve 42G and the solenoid valve 42H are opened and the other solenoid valves are closed to open the flow path from the first tank 31 through the second nozzle 43B to the pump 40. In this state, the control unit 5 drives the pump 40 to supply the cleaning liquid from the first tank 31 to the second nozzle 43B, thereby cleaning the electrode unit 222 of the sodium ion sensor 22. The cleaning liquid supplied to the second nozzle 43B is collected in the waste liquid tank 30.

Next, in step S111, the control unit 5 maintains the cleaning liquid supplied to the second nozzle 43B in the second nozzle 43B by closing the solenoid valve 42G. Then, in step S 112, the control unit 5 measures the voltage value VL with the sodium ion sensor 22 in a state in which the cleaning liquid is brought into contact with the electrode unit 222 of the sodium ion sensor 22. Then, the control unit 5 opens the solenoid valve 42G and closes the solenoid valve 42A, thereby delivering the cleaning liquid supplied to the second nozzle 43B to the waste liquid tank 30.

Next, in step S113, the control unit 5 opens the solenoid valve 42D, the solenoid valve 42G and the solenoid valve 42H and closes the other solenoid valves, to open the flow path from the fourth tank 34 through the second nozzle 43B to the pump 40. In this state, a medium concentration calibration solution for sodium ions is supplied from the fourth tank 34 to the second nozzle 43B by driving the pump 40, and then the electromagnetic valve 42G is closed to maintain the medium concentration calibration solution for sodium ions. Then, in step S114, the sodium ion sensor 22 measures the voltage value VM in a state in which the medium concentration calibration solution for sodium ions is brought into contact with the electrode unit 222 of the sodium ion sensor 22. Then, the control unit 5 opens the solenoid valve 42G and closes the solenoid valve 42D, to deliver the medium concentration calibration solution for sodium ions supplied to the second nozzle 43B to the waste liquid tank 30.

Next, in step S115, the control unit 5 opens the solenoid valve 42E, the solenoid valve 42G and the solenoid valve 42H and closes the other solenoid valves, to open the flow path from the fifth tank 35 through the second nozzle 43B to the pump 40. In this state, a high-concentration calibration solution for sodium ions is supplied from the fifth tank 35 to the second nozzle 43B by driving the pump 40, and thereafter the solenoid valve 42G is closed to maintain high concentration calibration solution for sodium ions to the second nozzle 43B. In step S116, the control unit 5 measures the voltage value VH with the sodium ion sensor 22 in a state in which the high concentration calibration solution for sodium ions is brought into contact with the electrode unit 222 of the sodium ion sensor 22. Then, the control unit 5 opens the solenoid valve 42G and closes the solenoid valve 42Eto deliver the high-concentration calibration solution for sodium ions supplied to the second nozzle 43B to the waste liquid tank 30.

Next, in step S117, the control unit 5 performs cleaning of the sodium ion sensor 22 in the same manner as in step S110.

Next, in step S118, the control unit 5 creates a calibration curve for sodium ions and store it in the storage unit based on the concentrations of the cleaning solution, medium concentration calibration solution for sodium ions and high concentration calibration solution for sodium ions, the measured voltage value VL, voltage value VM, and voltage value VH stored in the storage unit.

Next, in step S119, the control unit 5 controls the motor 2312 of the vertical movement drive unit 231 to raise the glucose sensor 21 and the sodium ion sensor 22 to the top dead center (step S120: YES), and move the sensors from the cleaning position to the standby position.

Returning to FIG. 23, when the calibration process of step S12 is completed, the control unit 5 stands by until a measurement start instruction is given in step S13, and when an instruction to start the measurement is issued, in step S 14 the control unit 5 confirms whether the interstitial fluid collector 110 and the like is installed in the setting unit 20. Upon receipt of the electric signal from the collector detecting sensor 8, the control unit 5 determines that the interstitial fluid collector 110 and the like are installed in the setting unit 20 in step S14, and proceeds to step S15, where the interstitial fluid collector 110 and the perspiration collector 111 are measured. On the other hand, when the electric signal is not received from the collector detecting sensor 8 in step S14, the control unit 5determines that the interstitial fluid collector 110 and the like are not installed in the setting unit 20, and proceeds to step S16 and displays an error message on the operation display unit 6.

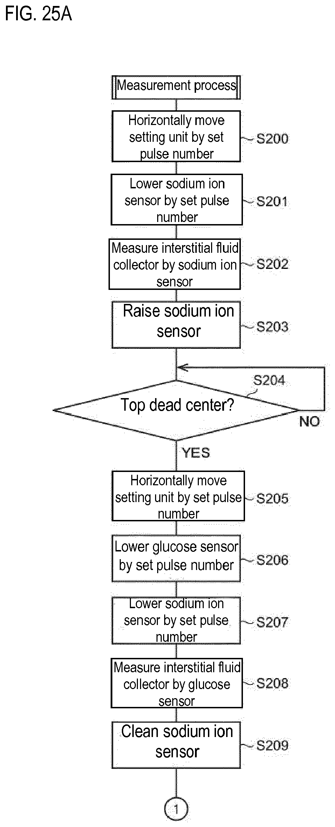

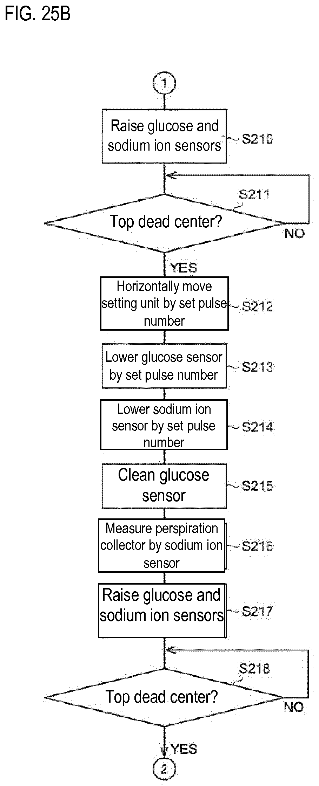

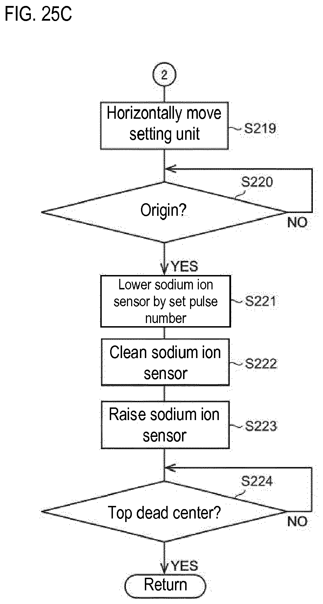

FIGS. 25A to 25C are flowcharts of the measurement process in step S15. First, in step S200, the control unit 5 controls the motor 2300 of the horizontal movement drive unit 230 to horizontally move the setting unit 20 by the set number of pulses, and moves the setting unit 20 from the installation position (shown in (a) of FIG. 17) to the first measurement position (shown in (b) of FIG. 17) where the interstitial fluid collector 110 is positioned below the sodium ion sensor 22. Then, in step S201, the control unit 5 controls the motor 2312 of the vertical movement drive unit 231 to lower the sodium ion sensor 22 by the set number of pulses, and moves the sodium ion sensor 22 from the standby position (shown in FIG. 20A) to the measurement position (shown in FIG. 20B), whereby sensor 22 is brought into contact with the interstitial fluid collector 110. Then, in step 202, the control unit 5 measures the voltage value VNaI of the interstitial fluid collector 110 by the sodium ion sensor 22. The voltage value VNaI depends on the sodium ion concentration CNaI in the interstitial fluid collected in the interstitial fluid collector 110. The measured voltage value VNaI is stored in the storage unit. In step S203, the control unit 5 controls the motor 2312 of the vertical movement drive unit 231 to raise the sodium ion sensor 22 to the top dead center (step S204: YES), and moves the sensor 22 from the measurement position to the standby position.

Next, in step S205, the control unit 5 controls the motor 2300 of the horizontal movement drive unit 230 so as to horizontally move the setting unit 20 by the set number of pulses to move the interstitial fluid collector 110 from the first measurement position to a second measurement position (shown in (c) of FIG. 17) located below the glucose sensor 21. Then, in step S206, the control unit 5 controls the motor 2312 of the vertical movement drive unit 231 to lower the glucose sensor 21 by the set number of pulses, to move from the standby position to the measurement position and is brought into contact with the interstitial fluid collector 110. Further, in step S207, the control unit 5 controls the motor 2312 of the vertical movement drive unit 231 to lower the sodium ion sensor 22 by the set number of pulses to move from the standby position to the cleaning position (shown in FIG. 20C). Then, in step S208, the control unit 5 applies a constant voltage to the interstitial fluid collector 110 by the glucose sensor 21 and measures the current value IGlu. The current value IGlu depends on the glucose concentration CGlu in the interstitial fluid collected in the interstitial fluid collector 110. The measured current value is stored in the storage unit. The control unit 5 also cleans the sodium ion sensor 22 in step S209. In step S210, the control unit 5 controls the motor 2312 of the vertical movement drive unit 231 to raise the glucose sensor 21 and the sodium ion sensor 22 to the top dead center step S211: YES) and the sensors 21 and 22 are moved from the cleaning position to the standby position.

Next, in step S213, the control unit 5 controls the motor 2300 of the horizontal movement drive unit 230 so as to horizontally move the setting unit 20 a set number of pulses to move the second interstitial fluid collector 110 from the second measurement position to the third measurement position (shown in FIG. 20D) located below the sodium ion sensor 22. Then, in step S211, the control unit 5 controls the motor 2312 of the vertical movement drive unit 231 to lower the sodium ion sensor 22 by the set number of pulses and move the sensor 22 from the standby position to the measurement position to perspiration and come into contact with the perspiration collector 111. In step S214, the control unit 5 also controls the motor 2312 of the vertical movement drive unit 231 to lower the glucose sensor 21 by the set number of pulses and to move the glucose sensor 21 from the standby position to the cleaning position. Then, the control unit 5 cleans the glucose sensor 21 in step S215. In step S 216, the control unit 5 measures the voltage value VNa2 of the perspiration collector 111 by the sodium ion sensor 22. This voltage value VNa2 depends on the sodium ion concentration CNa2 in perspiration collected in the perspiration collector 111. The measured voltage value VNa2 is stored in the storage unit. Then, in step S217, the control unit 5 controls the motor 2312 of the vertical movement drive unit 231 to raise the glucose sensor 21 and the sodium ion sensor 22 to the top dead center (step S218: YES), to move the sensors 21 and 22 from the cleaning position and from the measurement position to the standby position.

Next, in step S219, the control unit 5 controls the motor 2300 of the horizontal movement drive unit 230 to horizontally move the setting unit 20 to the origin (step S220: YES), and move the setting unit 20 from the third measurement position to the installation position. Then, in step S221, the control unit 5 controls the motor 2312 of the vertical movement drive unit 231 to lower the sodium ion sensor 22 by the set number of pulses to move the sensor 22 from the standby position to the cleaning position. Then, in step S222, the control unit 5 cleans the sodium ion sensor 22. Then, in step S223, the control unit 5 controls the motor 2312 of the vertical movement drive unit 231 to raise the sodium ion sensor 22 to the top dead center (step S224: YES), and moves the sensor 22 from the cleaning position to the standby position.

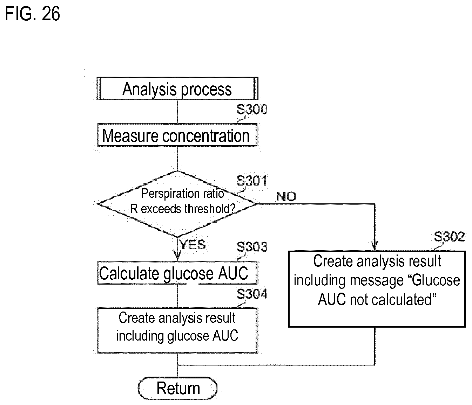

Returning to FIG. 23, when the measurement process in step S15 is completed, the control unit 5 analyzes each component collected in the interstitial fluid collector 110 and the perspiration collector 111 in step S17. FIG. 26 is a flowchart of the analysis process in step S17.

First, in step S300, the control unit 5 analyzes the glucose concentration CGlu and sodium concentration CNaI contained in the interstitial fluid collector 110 and the sodium ion concentration CNa2 collected in the perspiration collector 111 I do. Specifically, the control unit 5 first reads the glucose calibration curve (relational expression between the current value and the glucose concentration) from the storage unit, and applies the current value IGlu to the calibration curve based on the detection signal output from the glucose sensor 21 to calculate the glucose concentration CGlu. A sodium ion calibration curve (a relational expression between voltage value and sodium ion concentration) is read out from the storage unit, and a voltage value VNaI and a voltage value VNa2 are applied to the calibration curve based on the detection signal output from the sodium ion sensor 22 to calculate the sodium concentration CNal and the sodium ion concentration CNa2.

Next, in step S 301, the control unit 5 determines whether the perspiration rate R is equal to or more than a threshold value. The perspiration rate R is expressed by R = CNal / CNa2. When determining that the perspiration rate R is lower than the threshold value, the control unit 5 does not calculate the blood glucose AUC, and in step S302 the control unit 5 displays the analysis result of the blood glucose AUC because "the perspiration amount is large and the reliability of the analysis result can not be guaranteed. Please do not re-measure." In this way it is possible to avoid analysis of blood glucose AUC with low reliability. Note that the threshold value can be obtained from experimental data related to blood glucose AUC calculation value, blood glucose AUC due to blood collection and perspiration amount, which will be described later.

On the other hand, when determining that the perspiration rate R is equal to or greater than the threshold value in S301, the control unit 5 calculates the blood glucose AUC in S303. Specifically, the blood glucose AUC is calculated based on the glucose concentration CGlu, the sodium concentration CNal and the sodium concentration CNa2 obtained in step S300 and the following equation (1). Note that in the following equation (1), T is the sampling time of interstitial fluid. In this case $\alpha$ is the ratio of the transmittance between glucose and sodium ion and is a constant obtained by experiment. C'Na is the sodium ion concentration in blood and is a constant obtained by actual measurement.

$$AUC = C'Na \times T \times \{CGlu / \alpha (CNa1 -C CNa2)\} \ (1)$$

Then, in step S304, the control unit 5 generates an analysis result including the calculated blood glucose AUC. Analysis results can include glucose concentration, sodium concentration, perspiration rate and the like.

Returning to FIG. 23, in the next step S18, the control unit 5 displays the analysis result generated in step S17 on the operation display unit 6. Upon completion of the measurement of one interstitial fluid collector 110, the control unit 5 waits until there is an instruction to start measurement in step S13 or until the apparatus is shut down (YES in step S19).

As described above, in the present embodiment, when the interstitial fluid collector 110 is installed in the setting unit 20, the setting unit 20 moves to a measurement position separated from the installation position by a fixed distance, and the sensors 21 and 22 move from the standby position and are brought into contact with the interstitial fluid collector 110 by descending by a certain distance and moving to the measurement position. Therefore, it is possible to suppress variations in the positional relationship between the interstitial fluid collector 110 and the sensors 21 and 22 for each measurement, so that it is possible to stably obtain highly accurate measurement results.

In the present embodiment, when the measurement by the sodium ion sensor 22 is completed, a measurement is made by the glucose sensor 21 and the sodium ion sensor 22 is automatically cleaned during the measurement; and when the measurement by the glucose sensor 21 is completed, the next measurement by the sodium ion sensor 22 becomes possible and the glucose sensor 21 is automatically cleaned during the measurement by the sodium ion sensor 22 such that the next measurement becomes possible. In this way, since the cleaning of the sensors 21 and 22 is performed between the measurements, the cleaning of each of the sensors 21 and 22 can be performed efficiently, and the efficiency of the measurement work can be improved.

Other Modification Examples

Although the embodiments of the in-vivo component measuring apparatus and the in-vivo component measuring method have been described above, the present invention is not limited to the above-described embodiments and various modifications are possible insofar long as they do not deviate from the scope of the present invention. For example, the following changes are possible.

For example, in the above embodiment the drive unit 23 moves the setting unit 20 in the horizontal direction and moves the glucose sensor 21 and the sodium ion sensor 22 in the vertical direction, so that the setting unit 20 and each of the sensors 21 and 22 move to predetermined position and the glucose sensor 21 and the sodium ion sensor 22 are brought into contact with the interstitial fluid collector 110. However, movement is not limited to only the horizontal movement and the vertical movement, since the setting unit 20 and the sensors 21 and 22 also may be moved such that the movement direction of the setting unit 20 intersects with the movement direction of the sensors 21 and 22 to move the setting unit 20 and glucose sensor 21 and the sodium ion sensor 22 to predetermined positions whereby the sensors 21 and 22 are brought into contact with the interstitial fluid collector 110.

In the above-described embodiment, the drive unit 23 is configured to bring the glucose sensor 21 and the sodium ion sensor 22 into contact with the interstitial fluid collector 110 by moving both the setting unit 20, the glucose sensor 21, and the sodium ion sensor 22. However, the present invention is not limited thereto, and the drive unit 23 may bring the glucose sensor 21 and the sodium ion sensor 22 into contact with the interstitial fluid collector 110 by moving at least one of the setting unit 20, the glucose sensor 21, and the sodium ion sensor 22.

In the above embodiment, when the sensors 21 and 22 contact the interstitial fluid collector 110 and the perspiration collector 111, the contact pressure of the electrode units 212 and 222 is controlled by the pressure absorbing members 217 and 227 of the sensors 21 and 22, and the contact pressure of the electrode units 212 and 222 is adjusted by the pressure absorbing member 2014 of the setting unit 20. However, the contact pressure of the electrode units 212 and 222 also may be adjusted by using only one of the pressure absorbing members of the sensors 21 and 22 and the setting unit 20.

These pressure absorbing members 217, 227, and 2014 can be configured by elastic members other than spring members such as sponge and rubber.

In the above embodiment, when the sensors 21 and 22 are brought into contact with the interstitial fluid collector 110 or the perspiration collector 111, the cartridges 216 and 226 oscillate relative to the bodies 210 and 220 and adjust the angle of the surfaces of the electrode units 212 and 222 that contact the collector 110 and the perspiration collector 111. However, the present invention is not limited to this inasmuch as, for example, in the setting unit 20, the sample plate 200 may be oscillatably supported on the sample stage 201, and when the sensors 21 and 22 come into contact with the interstitial fluid collector 110 or the perspiration collector 111, the sample plate 200 is oscillated so as to adjust the angle of the surface of the interstitial fluid collector 110 and the surface of the perspiration collector 111 supported on the sample plate 200 so that the electrode units 212 and 222 can be brought into contact with the surfaces of the interstitial fluid collector 112 and the perspiration collector 111.

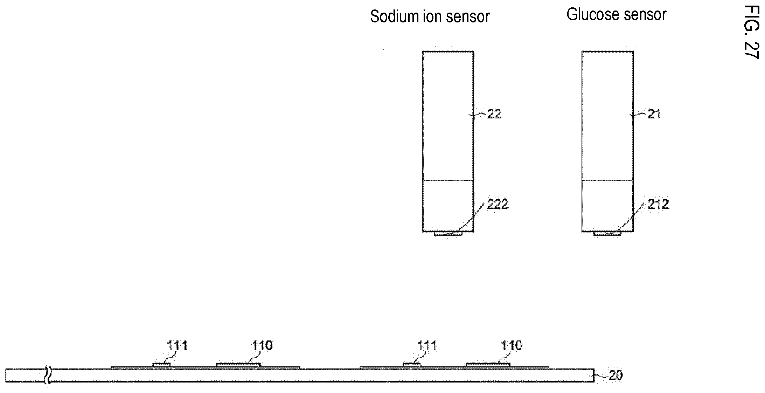

In the above embodiment, only one set of the interstitial fluid collector 110 and the perspiration collector 111 is installed for measurement in the setting unit 20 of the in-vivo component measurement apparatus 1. However, as shown in FIG. 27, a plurality of sets of the interstitial fluid collector 110 and the perspiration collector 111 can be installed in the setting unit 20, and a plurality of sets of the interstitial fluid collector 110 and the second collector 111 can be measured. In the modification of FIG. 27, the measurement process shown in FIGS. 25A to 25C is repeatedly performed for each set of the interstitial fluid collector 110 and the perspiration collector 111, and in step S17 of FIG. 23, and an analysis process shown in FIG. 26 is performed a blood glucose AUC is calculated for each set of the body 110 and the perspiration collector 111. According to the modified example of FIG. 27, when there are a plurality of sets of the interstitial fluid collector 110 and the perspiration collector 111 to be measured, it is not necessary for the operator of the apparatus to wait in the vicinity of the apparatus each time a measurement is carried out since it is unnecessary to manually set the interstitial fluid collector 110 and the set of the perspiration collector 111 in the setting unit 20 for each measurement of the sets of the interstitial fluid collector 110 and the perspiration collector 111, thereby improving the efficiency of the measurement work.

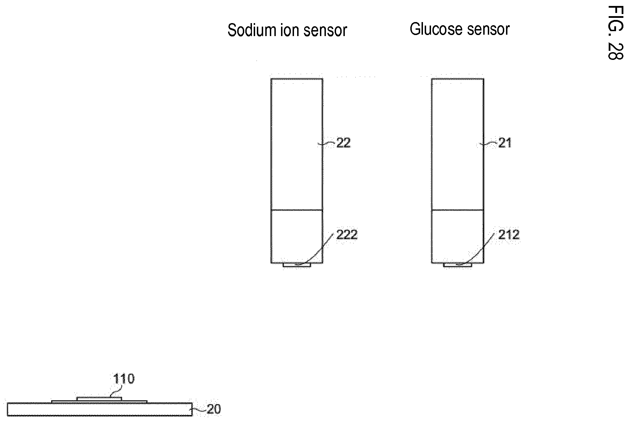

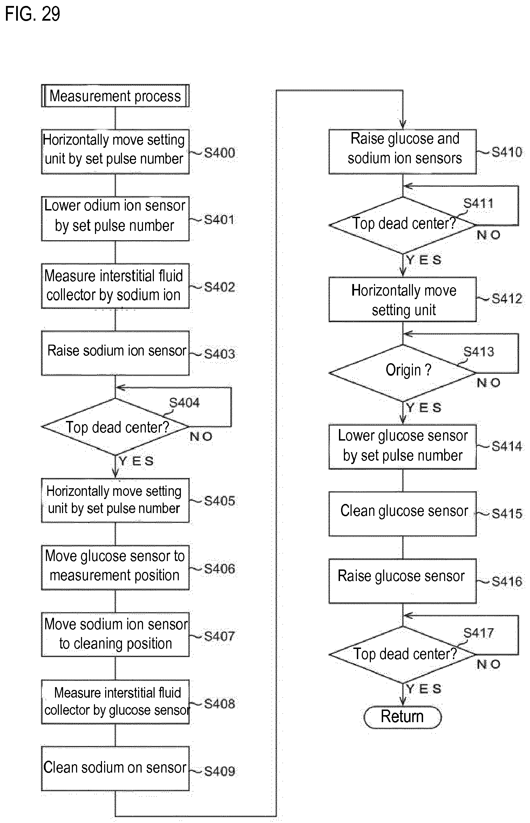

In the above embodiment, the interstitial fluid collector 110 and the perspiration collector 111 are installed in the setting unit 20 and measured by the in-vivo component measuring apparatus 1. However, the present invention is not limited to this, inasmuch as only the interstitial fluid collector 110 may be installed in the setting unit 20 for measurement, as shown in FIG. 28. In the modification of FIG. 28, the measurement process shown in FIG. 29 is performed in step S15 of FIG. 23, and the control unit 5 controls the motor 2300 of the horizontal movement drive unit 230 in steps S400 to S402, to move the setting unit 20 horizontally by the set number of pulses from the installation position to the first measurement position, and controls the motor 2312 of the vertical movement drive unit 231 so that the sodium ion sensor 22 is moved by the set number of pulses from the standby position to the measurement position where the voltage value VNaI of the interstitial fluid collector 110 is measured by the sodium ion sensor 22. Then, in steps S403 to S405, the control unit 5 controls the motor 2312 of the vertical movement drive unit 231 to raise the sodium ion sensor 22 to the top dead center from the measurement position to the standby position, and controls the motor 2300 of the drive unit 230 to horizontally move the setting unit 20 by the set number of pulses from the first measurement position to the second measurement position.

Next, in steps S406 to S409, the control unit 5 controls the motor 2312 of the vertical movement drive unit 231 to lower the glucose sensor 21 by the set number of pulses from the standby position to the measurement position, and the motor 2312 of the vertical movement drive unit 231 is controlled to lower the sodium ion sensor 22 by the set number of pulses from the standby position to the cleaning position, then the current value IGlu of the interstitial fluid collector 110 is measured by the sensor 21 and the sodium ion sensor 22 is cleaned. Then, in steps S410 to S413, the control unit 5 controls the motor 2312 of the vertical movement drive unit 231 to raise the glucose sensor 21 and the sodium ion sensor 22 to the top dead center from the measurement position and the cleaning position, and controls the motor 2300 of the horizontal movement drive unit 230 to horizontally move the setting unit 20 to the origin, that is, from the second measurement position to the installation position.

Next, in steps S414 to S417, the control unit 5 controls the motor 2312 of the vertical movement drive unit 231 to lower the glucose sensor 21 by the set number of pulses from the standby position to the cleaning position, and after cleaning the glucose sensor 21, the motor 2312 of the vertical movement drive unit 231 is controlled to raise the glucose sensor 21 to the top dead center from the cleaning position to the standby position.

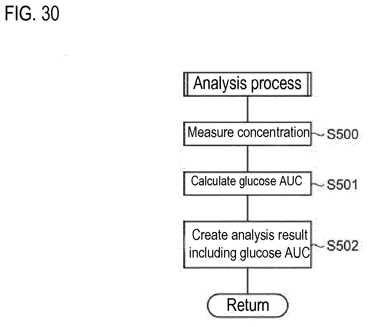

Then, in the step S17 of FIG. 23, the analysis process shown in FIG. 30 is performed, and the control unit 5 analyzes the glucose concentration CGlu and the sodium concentration CNal contained in the interstitial fluid collected in the interstitial fluid collector 110 in step S500; in step S501 the blood glucose AUC is calculated based on the equation (2) below and the glucose concentration CGlu and the sodium concentration CNal obtained in step S500, and in step S502 an analysis result including the calculated blood glucose AUC is created.

$$AUC = C'Na \times T \times (CGlu / \alpha CNa1) \ (2)$$

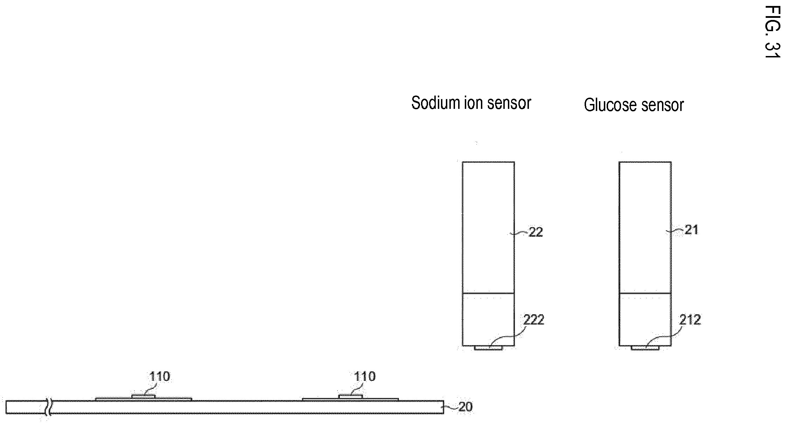

Note that in the modified example of FIG. 28, only one interstitial fluid collector 110 is installed and measured in the setting unit 20 of the in-vivo component measuring apparatus 1. However, the present invention is not limited to this, as shown in FIG. 31, a plurality of interstitial fluid collectors 110 may be installed in the setting unit 20, and the plurality of interstitial fluid collectors 110 installed in the setting unit 20 may be measured. In the modification of FIG. 31, the measurement process shown in FIG. 29 is repeatedly performed for each interstitial fluid collector 110 in step S15 of FIG. 23, and in step S17 of FIG. 23 analysis processing to calculate blood glucose AUC is performed for each interstitial fluid collector 110. According to the modification of FIG. 31, since there is no need to manually place the interstitial fluid collector 110 in the setting unit 20 for each measurement of the interstitial fluid collector 110 when there are a plurality of interstitial fluid collectors 110 to be measured, the operator does not have to wait at the side of the apparatus during every measurement and it is possible to improve the efficiency of the measurement work.

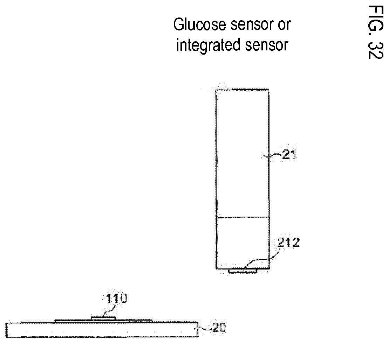

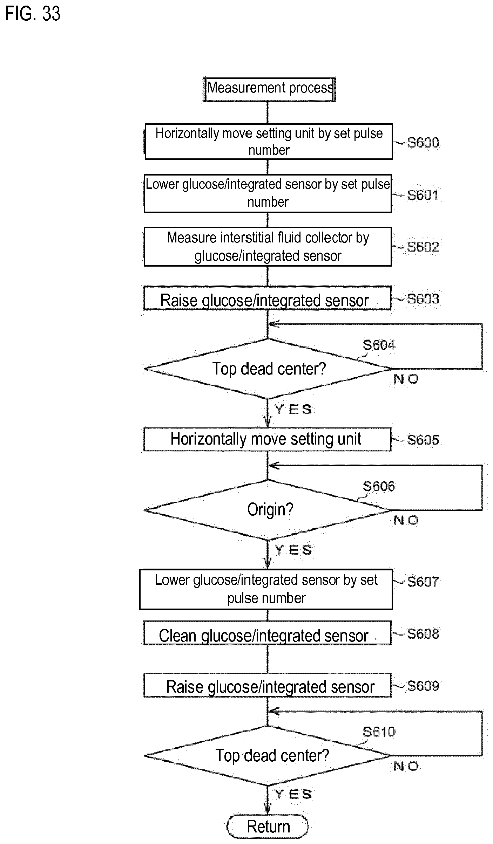

In the modification of FIG. 28, the interstitial fluid collector 110 also is measured using the two acquiring units 21 and 22 of a glucose sensor and a sodium ion sensor. However, the invention is not limited to this inasmuch as measurement of the interstitial fluid collector 110 may be performed using only one acquiring unit 21, as shown in FIG. 32. In the modification of FIG. 32, the acquiring unit 21 is a glucose sensor that measures a signal reflecting the amount of glucose, and the measurement process shown in FIG. 33 is performed in step S15 of FIG. 23. Specifically, in step S600, the control unit 5 first controls the motor 2300 of the horizontal movement drive unit 230 to horizontally move the setting unit 20 by the set number of pulses from the installation position to the measurement position where the interstitial fluid collector 110 is located below the glucose sensor 21.

Then, in step S601, the control unit 5 controls the motor 2312 of the vertical movement drive unit 231 to lower the glucose sensor 21 by the set number of pulses from the standby position to the measurement position so as to contact the collector 110. Then, in step 602, the control unit 5 applies a constant voltage to the interstitial fluid collector 110 by the glucose sensor 21 and measures the current value IGlu related to the glucose concentration CGlu in the interstitial fluid. Then, in steps S603 to S604, the control unit 5 controls the motor 2312 of the vertical movement drive unit 231 to move the glucose sensor 21 to the top dead center from the measurement position to the standby position, and then in step S605 to step S606, the motor 2300 of the horizontal movement drive unit 230 is controlled to horizontally move the setting unit 20 to the origin, that is, from the measurement position to the installation position. Then, in steps S608 to S611, the control unit 5 controls the motor 2312 of the vertical movement drive unit 231 to lower the glucose sensor 21 by the set number of pulses from the standby position to the cleaning position, and after cleaning the glucose sensor 21, the motor 2312 of the vertical movement drive unit 231 is controlled to raise the glucose sensor 21 to the top dead center, that is, from the cleaning position to the standby position.

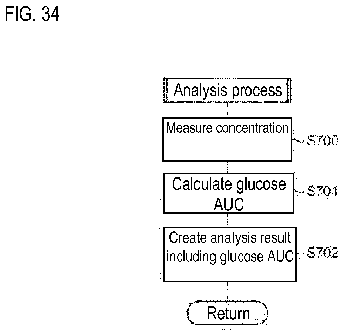

Then, in step S17 of FIG. 23, the analysis process shown in FIG. 34 is performed, and the control unit 5 analyzes the glucose concentration CGlu contained in the interstitial fluid collected in the interstitial fluid collector 110 in step S700, then in step S701 blood glucose AUC is calculated based on the glucose concentration CGlu obtained in step S700 by the following equation (3), and in step 702 an analysis result including the calculated blood glucose AUC is generated. Note that in the following equation (3), $\beta$ is a constant obtained by experiment.

$$AUC = \beta \ CGlu \ (3)$$

Note that although the acquiring unit 21 is a glucose sensor in the modification of FIG. 32, the acquiring unit 21 also may be an integrated sensor. The integrated sensor is capable of measuring a signal reflecting the amount of glucose and a signal reflecting the amount of sodium ions. When the acquiring unit 21 is an integrated sensor, the integrated sensor includes a first electrode unit for glucose measurement and a second electrode unit for sodium ion measurement as electrode unit units to be in contact with the interstitial fluid collector 110. The integrated sensor also includes a glucose measuring circuit connected to the first electrode unit and a sodium ion measuring circuit connected to the second electrode unit as an electric circuit.

In this modification, in the measurement process shown in FIG. 33 in step S602, the control unit 5 applies a constant voltage to the interstitial fluid collector 110 by the integrated sensor 21 to obtain the current value IGlu related to the glucose concentration CGlu in the interstitial fluid, and after the glucose measurement, the voltage value VNaI related to the sodium ion concentration CNaI in the interstitial fluid of the interstitial fluid collector 110 is measured by the integrated sensor 21. Then, in step S17 of FIG. 23, the above-described analysis process shown in FIG. 30 is performed, and the blood glucose AUC is calculated using the above equation (2). According to this modification, it is possible to calculate the blood glucose AUC by analyzing the concentration of sodium ions as an auxiliary component together with the concentration of glucose which is a measurement target component in one acquiring unit, so that the efficiency and simplification of measurement work can be achieved.

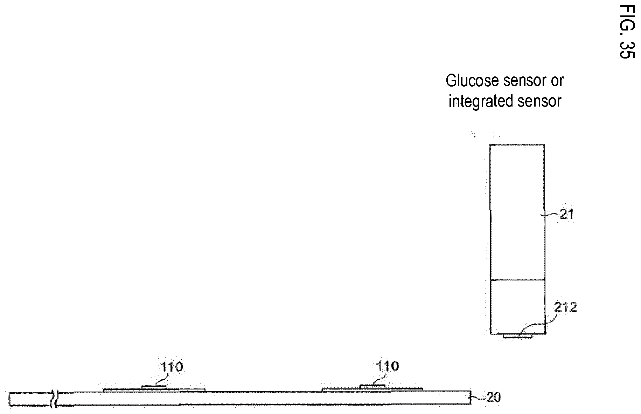

In the modified example of FIG. 32, only one interstitial fluid collector 110 is installed and measured in the setting unit 20 of the in-vivo component measurement apparatus 1. However, the invention is not limited to this, inasmuch as a plurality of interstitial fluid collectors 110 may be installed in the setting unit 20, and the plurality of interstitial fluid collectors 110 installed in the setting unit 20 may be measured, as shown in FIG. 35. In the modification of FIG. 35, the measurement process shown in FIG. 33 is repeatedly performed for each interstitial fluid collector 110 in step S15 of FIG. 23; in step S17 of FIG. 23 the analysis process of FIG. 34 is performed for each interstitial fluid collector 110 (when the acquiring unit 21 is a glucose sensor) or the analysis process of FIG. 30 is performed for each interstitial fluid collector 110 (when the acquiring unit 21 is an integrated sensor) to calculate blood glucose AUC. Even in the modification of FIG. 35, it is possible to improve the efficiency of measurement work when there are a plurality of interstitial fluid collectors 110 to be measured.

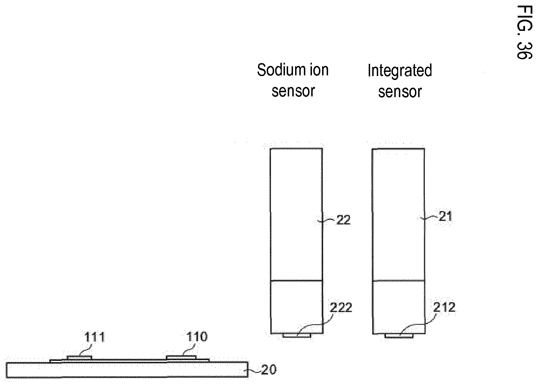

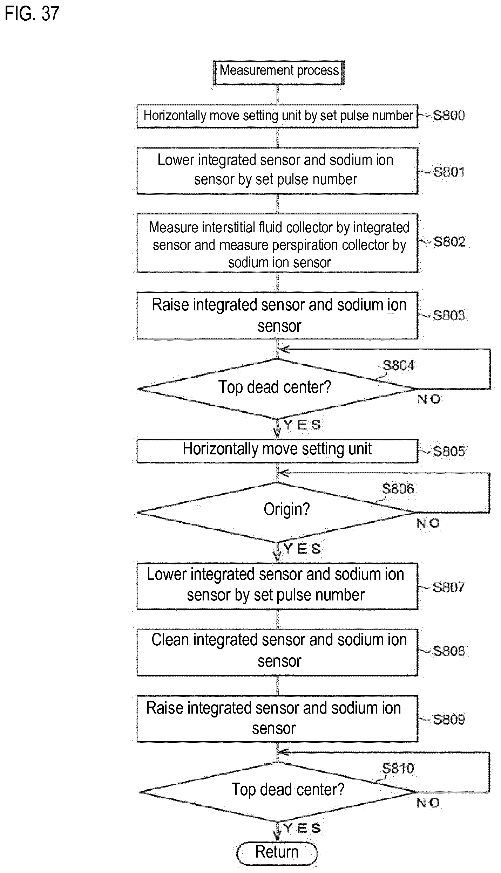

In the above embodiment, the acquiring unit 21 also may be an integrated sensor as shown in FIG. 36. In this modification, the measurement process shown in FIG. 37 is performed in step S15 of FIG. 23, and in step S800 the control unit 5 controls the motor 2300 of the horizontal movement drive unit 230 to move the setting unit 20 horizontally for the set number of pulses from the installation position to the measurement position where the interstitial fluid collector 110 is positioned below the acquiring unit 21 and the perspiration collector 111 is positioned below the sodium ion sensor 22. Then, in step S801, the control unit 5 controls the motor 2312 of the vertical movement drive unit 231 to lower the integrated sensor 21 and the sodium ion sensor 22 by the set number of pulses from the standby position to the measurement position so as to be brought into contact with the interstitial fluid collector 110 and the perspiration collector 111, respectively.

Then, in step 802, the control unit 5 applies a constant voltage to the interstitial fluid collector 110 by the integrated sensor 21 to measure the current value IGlu related to the glucose concentration CGlu in the interstitial fluid, and then the integral sensor 21 measures a voltage value VNaI related to the sodium ion concentration CNaI in the interstitial fluid of the interstitial fluid collector 110. At the same time, the sodium ion sensor 22 measures the voltage value VNa2 related to the sodium ion concentration CNaI in the perspiration of the perspiration collector 111. Then, in steps S803 to S804, the control unit 5 controls the motor 2312 of the vertical movement drive unit 231 to raise the integrated sensor 21 and the sodium ion sensor 22 to the top dead center from the measurement position to the standby position, and in steps S805 to S806, the motor 2300 of the horizontal movement drive unit 230 is controlled to horizontally move the setting unit 20 to the origin from the measurement position to the installation position. Then, in steps S807 to S810, the control unit 5 controls the motor 2312 of the vertical movement drive unit 231 to lower the integral type sensor 21 and the sodium ion sensor 22 by the set number of pulses from the standby position to the cleaning position; and after cleaning the integrated sensor 21 and the sodium ion sensor 22, the motor 2312 of the vertical movement drive unit 231 is controlled to move the integrated sensor 21 and the sodium ion sensor 22 to the top dead center, that is, from the cleaning position to the standby position.

Then, in step S17 of FIG. 23, the above-described analysis process shown in FIG. 26 is performed, and the blood glucose AUC is calculated using the above equation (1).

According to the modification of FIG. 36, the interstitial fluid collector 110 and the perspiration collector 111 can be measured at the same time, and the acquisition unit 21 and the sodium ion sensor 22 can be cleaned at the same time, thereby improving efficiency and simplifying the measurement work.

Note that in the modification of FIG. 36 a plurality of sets of the interstitial fluid collectors 110 and the perspiration collectors 111 can be installed in the setting unit 20, and a plurality of sets of interstitial fluid collectors 110 and the sets of the perspiration collectors 111 may be measured. In this modification, the measurement process shown in FIG. 37 is repeated for each set of the interstitial fluid collector 110 and the perspiration collector 111 in step S15 of FIG. 23, and in step S17 of FIG. 23 the analysis process shown in FIG. 26 is performed for each set of the interstitial fluid collectors 110 and second collectors 11 to calculate the blood glucose AUC. In this modified example, when there are a plurality of sets of the interstitial fluid collector 110 and the perspiration collector 111 to be measured, it also is possible to improve the efficiency of the measurement work.

In the above-described embodiment and modifications, the sodium ion sensor 22 also acquires the voltage values of the interstitial fluid collector 110 and the perspiration collector 111 in order to measure the concentration of sodium ions contained in the interstitial fluid or perspiration. However, the invention is not limited to this, inasmuch as the sodium ion sensor 22 also may acquire the current value by applying a constant voltage to the interstitial fluid collector 110 or the perspiration collector 111.

In the above embodiment and each modification, the blood glucose AUC also is calculated by substituting the glucose concentration and the sodium ion concentration into the above equations (1) to (3). However, the present invention is not limited thereto, and the blood glucose AUC can be calculated using various known calculation methods.

In the above-described embodiment and modifications, the interstitial fluid collector 110 also is directly fixed to the skin of the subject to collect interstitial fluid. However, the present invention is not limited to this, inasmuch as the skin of a subject may be covered with a film or sheet that prevents permeation of perspiration, and the skin may be pierced so as to penetrate the film or sheet with the puncture tool 100 from above the film or sheet. By collecting interstitial fluid from the plurality of micropores formed in the skin through the film or sheet with the interstitial fluid collector 110, perspiration from the skin is restricted from moving to the interstitial fluid collector 110 by the film or sheet. In this way it is possible to prevent perspiration from mixing in the interstitial fluid at the time of collecting the interstitial fluid by the interstitial fluid collector 110 and accumulating perspiration-derived sodium ions in the interstitial fluid collector 110, hence, when calculating the blood glucose AUC the effect of perspiration can be avoided. Therefore, even without using the perspiration collector 111, it is possible to calculate the blood glucose AUC with high reliability, and it is possible to improve the efficiency and simplification of the measurement work. Note that, for example, the film described in Japanese Patent Application Publication No. 2012-217667 can be used as the film or sheet.

In the above-described embodiment and each modification, the calibration is performed at the time of activation of the apparatus. However, the present invention is not limited to this, and the calibration may be performed as necessary; for example, the calibration may be performed each time measurement of one interstitial fluid collector 110 is completed, or the calibration may be performed for a predetermined number of interstitial fluid collectors 110.

In the above embodiment and modifications, a refresh process for recovering a decrease in sensitivity also may be performed when the sensitivity of the electrode unit 212 of the glucose sensor 21 decreases. For example, by applying a potential opposite to the measurement potential applied at the time of measurement to the working electrode of the electrode unit 212 in a pulsed manner, the electrode unit 212 is refreshed to restore the detection sensitivity of glucose by the electrode unit 212.

In the above-described embodiment and modifications, pretreatment for suppressing reduction in sensitivity of the electrode unit 212 of the glucose sensor 21 also may be performed. For example, by repeatedly applying a potential higher than the measurement potential applied at the time of measurement and a potential lower than the measurement potential to the working electrode of the electrode unit 212 for a predetermined time period, the glucose sensor 21 the detection of glucose by the electrode section 212 can be maintained with high sensitivity even when used for a long period of time.

Verification of Effect of Pretreatment

Fabrication of Glucose Sensor

(1) Preparation of Electrode

On a ceramic substrate, a working electrode composed of platinum paste with a diameter of 5 mm and a counter electrode, and a reference electrode made of silver/silver chloride paste were formed by a screen printing method.

(2) Formation of Immobilized Enzyme Membrane

To 12.7 μL of phosphate buffered saline (PBS) were added 6.8 μL of glucose oxidase (GOD) solution, 6.8 μL of mutarotase (MUT) solution, 7.8 μL of BSA solution, and finally 4.9 μL of GA solution was added and stirred to prepare BSA (214.92 mg/dL) · glucose oxidase (0.87 U/μL) · GA (2%) · mutarotase (1 U/mL) solution. The working electrode was coated with 0.57 μL of the above solution. In this state, electrodes were placed in a petri dish, and were allowed to stand for 24 hours in a thermostatic chamber (Espec, SH-221, Japan) set at a temperature of 25°C, and humidity of 30%, and the solution on the working electrode was dried to form a membrane on the working electrode to prepare a glucose sensor. The glucose sensor also was stored at 4°C until use.

Pretreatment Procedure

Each electrode of the glucose sensor was connected to a potentiostat, and 150 μL of a 268 mM potassium chloride aqueous solution was dripped so as to cover the three poles, and a potential was applied to the working electrode and scanned by the method shown in Table 1.

Table 1

| | Pretreatment Method | Pretreatment time |
|---|---|---|
| Example 1 | Apply 0.6 V ⇔ 1.0 V every 20 seconds for each potential 20 times | 40 sec |
| Example 2 | Apply 0.6 V ⇔ 1.0 V every 20 seconds for each potential 20 times | 40 sec |
| Example 3 | Apply 0.6 V ⇔ 1.0 V every 20 seconds for each potential 20 times | 80 sec |
| Comparative example 1 | Successive submotion method (triangular wave potential scanning) 10 reciprocations at a scanning speed of 0.1 V/s between -1.0 and 1.0 V | 410 sec |
| Comparative example 2 | Successive submotion method (triangular wave potential scanning) 10 reciprocations at a scanning speed of 0.1 V/s between -1.0 and 1.0 V | 810 sec |
| Comparative example 3 | Successive submotion method (triangular wave potential scanning) 10 reciprocations at a scanning speed of 0.1 V/s between -1.0 and 1.0 V | 1610 sec |

Electrochemical Measurement

After performing the pretreatment described above, the glucose sensor was connected to the potentiostat, 150 $\mu$L of the PB-K solution was dripped so as to cover the three poles, and a voltage of 0.45 V was applied to the working electrode with the reference electrode as reference. Thereafter, the sensor was allowed to stand for 600 seconds. The PB-K solution was removed, 150 $\mu$L of 1 mg/dL glucose · PB-K solution was added drop-wise, and the mixture was allowed to stand for 3 minutes. Thereafter, the above solution was removed, 150 $\mu$L of a 5 mg/dL glucose • PB-K solution was added drop-wise, and the mixture was allowed to stand for 3 minutes. Again, the above solution was removed, 150 $\mu$L of a 40 mg/dL glucose • PB-K solution was added drop-wise and left to stand for 3 minutes. Finally, the above solution was removed, and the glucose sensor was washed by repeating dripping, removing, and dripping 150 $\mu$L of PB-K solution. This series of operations was carried out in a state where a voltage of 0.45 V was applied and variation of the current value during that time was recorded. In addition, the current value for the glucose sensor in each concentration solution was set as the average value for 60 seconds from 120 seconds after the solution of each concentration was dripped.

Results

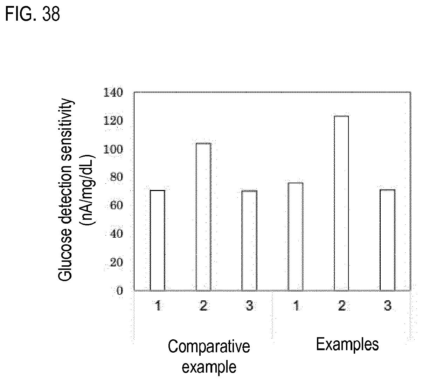

FIG. 38 shows the detection sensitivity of glucose sensor subjected to the above-described pretreatment to glucose. In Comparative Examples 1 to 3, the detection sensitivities are 70.5, 103.9 and 70.3 nA/mg/dL, respectively, and the pretreatment times are 410 seconds, 810 seconds, and 1610 seconds, respectively. In Examples 1 to 3, the detection sensitivities are 75.9, 122.0, and 70.9 nA/mg/dL, respectively, and the pretreatment times are 40 seconds, 40 seconds, and 80 seconds, respectively. From FIG. 38, it can be seen that in Examples 1 to 3, the detection sensitivity of the glucose sensor can achieve high sensitivity in a short time by performing the pretreatment.

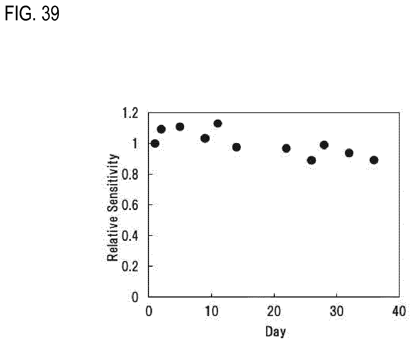

FIG. 39 also shows the relative detection sensitivity with the first day being 1 for the glucose sensor subjected to the pretreatment described above. It is understood from FIG. 39 that the detection sensitivity of the glucose sensor can be kept high for a long period by performing the above-described pretreatment, and the glucose sensor can continuously be measured over a long term.

Although an example in which a plurality of micropores are formed in the skin of a subject using the puncture tool 100 to promote exudation of the interstitial fluid has been described in the above embodiment, the present invention is not limited to this example, inasmuch as various known methods such as promoting exudation of interstitial fluid by so-called peeling or the like can be used.

In the above embodiment, the control unit 5 functions as a control unit that controls the drive unit 23 of the detecting unit 2, and also functions as an analysis unit that measures glucose concentration and sodium ion concentration based on a signal reflecting the amount of measurement target component (glucose) and a signal reflecting the amount of electrolyte (sodium ion) as an auxiliary component by the sensors 21 and 22 of the detecting unit 2, and calculates blood glucose AUC. However, the present invention is not limited to this, and apart from the control unit 5 that controls the drive unit 23, an analysis device such as a computer that calculates blood glucose AUC may be provided. This analysis device may be, for example, a server device on the cloud connected to the control unit 5 via a network, such that the control unit 5 transmits each signal acquired by each of the sensors 21 and 22 to the analysis device, and the analysis device calculates blood glucose AUC based on each received signal, and generates an analysis result including blood glucose AUC, and the control unit 5 may display the analysis result on the operation display unit 6 by receiving the analysis result from the analysis device.

In the above embodiment, sodium ions are measured as the electrolyte in the interstitial fluid, but the present invention is not limited to this, inasmuch as inorganic ions such as potassium ion, calcium ion, magnesium ion, zinc ion, chloride ion, and the like also may be measured.

Although an example of calculating the blood glucose AUC is described in the above embodiment, the calculated value is not limited to blood glucose AUC and another value may be calculated insofar as it is a value corresponding to the in-vivo concentration of glucose.

Although an example in which glucose in the interstitial fluid is measured is described in the above embodiment, the present invention is not limited to this example, and components other than glucose contained in the interstitial fluid also may be measured. Examples of components measured according to the present invention include biochemical components and drugs administered to a subject. Examples of biochemical components include albumin, globulin, and enzymes of proteins which are one type of biochemical component. In addition, creatinine, creatine, uric acid, amino acids, fructose, galactose, pentose, glycogen, lactic acid, caffeine, pyruvic acid, ketone bodies and the like can be mentioned as biochemical components other than protein. Examples of the drugs include digitalis preparations, theophylline, drugs for arrhythmia, antiepileptics, amino acid sugar antibiotics, glycopeptide antibiotics, antithrombotics and immunosuppressants.

**Claims**

1. An in-vivo component measuring apparatus for measuring components contained in interstitial fluid collected from a subject, comprising:

   a setting unit in which a collector that collects the interstitial fluid is installed;
   an acquiring unit that acquires a signal that reflects an amount of a measurement target component contained in the interstitial fluid when in a state of contact with the collector installed in the setting unit; and
   a moving unit that brings the collector installed in the setting unit into contact with the acquiring unit by changing a relative position between the setting unit and the acquiring unit to a predetermined positional relationship.

2. The in-vivo component measuring apparatus according to claim 1, further comprising:
   an analyzing unit that generates a value related to the amount of the measurement target component based on the signal reflecting the amount of the measurement target component acquired by the acquiring unit.

3. The in-vivo component measuring apparatus according to claim 1 or 2, wherein
   the moving unit changes the relative position between the setting unit and the acquiring unit to the predetermined positional relationship by moving the acquiring unit relative to the setting unit to a predetermined position.

4. The in-vivo component measuring apparatus according to claim 3, wherein
   the moving unit moves the acquiring unit in a vertical direction with respect to the setting unit.

5. The in-vivo component measuring apparatus according to claim 3 or 4, further comprising:

   a position detecting sensor that detects a position of the acquiring unit;
   wherein the moving unit moves the acquiring unit to the predetermined position based on a detection signal from the position detecting sensor.

6. The in-vivo component measuring apparatus according to claim 5, wherein
   the position detecting sensor detects a reference position of the acquiring unit; and
   the moving unit moves the acquiring unit to the predetermined position by moving the acquiring unit a predetermined distance from the reference position.

7. The in-vivo component measuring apparatus according to any one of claims 1 to 6, wherein
   while the acquiring unit acquires the signal reflecting the amount of the measurement target component, the moving unit maintains a state in which the acquiring unit is pressed against the collector installed in the setting unit.

8. The in-vivo component measuring apparatus according to any one of claims 1 to 7, wherein

the moving unit includes a pressure regulator that regulates a contact pressure so as to be constant when the acquiring unit is in contact with the collector.

9. The in-vivo component measuring apparatus according to claim 8, wherein
the pressure regulator includes a pressure absorbing member provided in the acquiring unit or the setting unit.

10. The in-vivo component measuring apparatus according to any one of claims 1 to 9, wherein
the moving unit comprises an angle adjuster that adjusts an angle at which the acquiring unit makes contact with the collector.

11. The in-vivo component measuring apparatus according to any one of claims 3-6, wherein
the moving unit moves the setting unit to a measurement position and moves the acquiring unit to the predetermined position with respect to the setting unit at the measurement position to change the relative position between the setting unit and the acquiring unit to the predetermined positional relationship.

12. The in-vivo component measuring apparatus according to any one of claims 1 to 11, wherein
the setting unit includes a positioning unit that positions the collector.

13. The in-vivo component measuring apparatus according to anyone of claims 1 to 12, wherein
the setting unit is configured to hold a plurality of collectors; and
the moving unit brings the acquiring unit into contact with each of the collectors held by the setting unit.

14. The in-vivo component measuring apparatus according to anyone of claims 1 to 13, further comprising:

a tank that stores a cleaning liquid; and a pump that delivers the cleaning liquid from the tank;
wherein the moving unit moves the setting unit from a measuring position after the collector is measured by the acquiring unit; and
the pump delivers the cleaning liquid to the acquiring unit to perform cleaning.

15. An in-vivo component measuring method for measuring a component contained in interstitial fluid collected from a subject, comprising steps of:

changing a relative position between a component detection sensor and a setting unit in which is installed a collector that collects interstitial fluid to a predetermined positional relationship;
bringing the component detection sensor into contact with the collector installed in the setting unit under a constant pressure while in the predetermined positional relationship;
acquiring a signal reflecting an amount of a measurement target component contained in the interstitial fluid by the component detection sensor that is in a state of contact with the collector; and
generating a value related to the amount of the measurement target component based on the acquired signal reflecting the amount of the measurement target component.

FIG. 1

FIG. 2

FIG. 3

FIG. 4

S

H H H H H

Stratum corneum
Stratum granulosa } Epidermis

Dermis

Subcutaneous
tissue

FIG. 5A

112    113

111    110

A    A

FIG. 5B

111    110

113    112

FIG. 6A

FIG. 6B

FIG. 6C

FIG. 7A

FIG. 7B

FIG. 8A

FIG. 8B

FIG. 9

FIG. 10A

2001

200

2002A

B

B

2003

2001

2000

2002B

FIG. 10B

2004

2003

2002B

2002A

2001

200

2005

FIG. 10C

2001

2000

2002A

2003

2005

200

FIG. 11A

FIG. 11B

FIG. 12A

D ◄

E ↑

E ↑

2013

201

2010   2014(61)   2011

D ◄

FIG. 12B

2013

2012

2013

2012

2010   201

FIG. 12C

2014(61)   2012

2010   2011   201

FIG. 13A

FIG. 13B

FIG. 14A

FIG. 14B

FIG. 15

FIG. 16

FIG. 17

(sodium ion sensor)

(Glucosesensor)

(a) 1st Installation position

(b) 1st measurement position

(c) 2nd measurement position

(d) 3rd measurement position

FIG. 18A

2312    231    19A(19)    21,22    19B(19)

M    90    91    9    2311    2310    2313    24    92

FIG. 18B

24    9    91    92    2310    2313    2312    M    2311    231    21,22    212,222    19A(19)    19B(19)

FIG. 19A

FIG. 19B

FIG. 20

(a) Standby position    (b) Measurement position    (c) Cleaning position

FIG. 21

FIG. 22

FIG. 23

START

S10
Power on? —— NO

YES
S11
Initialization process

S12
Calibration process

S13
Start measurement instruction? —— NO

YES

S14
Interstitial fluid collector installed in setting unit? —— NO

YES
S15
Measurement process

S16
Error display

S17
Analysis process

S18
Display analysis result

S19
Shutdown? —— NO

YES
END

FIG. 24

Calibration process

Lower glucose and sodium ion sensors by set pulse number — S100

Clean glucose sensor — S101

Supply cleaning liquid to 1st nozzle — S102

Measure current by glucose sensor — S103

Supply low concentration calibration solution to 1st nozzle — S104

Measure current by glucose sensor — S105

Supply high concentration calibration solution to 1st nozzle — S106

Measure current by glucose sensor — S107

Clean glucose sensor — S108

Create glucose calibration curve — S109

Clean sodium ion sensor — S110

Supply cleaning liquid to 2nd nozzle — S111

Measure current by sodium ion sensor — S112

Supply medium concentration calibration solution to 2nd nozzle — S113

Measure current by sodium ion sensor — S114

Supply high concentration calibration solution to 2nd nozzle — S115

Measure current by sodium ion sensor — S116

Clean sodium ion sensor — S117

Create sodium ion calibration curve — S118

Raise glucose and sodium ion sensors — S119

Top dead center? — S120

NO

YES

Return

49

FIG. 25A

```
┌─────────────────────────┐
│ Measurement process     │
└─────────────────────────┘
            │
┌─────────────────────────┐
│ Horizontally move        │
│ setting unit by set      │ ~S200
│ pulse number             │
└─────────────────────────┘
            │
┌─────────────────────────┐
│ Lower sodium ion         │
│ sensor by set pulse      │ ~S201
│ number                   │
└─────────────────────────┘
            │
┌─────────────────────────┐
│ Measure interstitial fluid│
│ collector by sodium ion  │ ~S202
│ sensor                   │
└─────────────────────────┘
            │
┌─────────────────────────┐
│ Raise sodium ion         │
│ sensor                   │ ~S203
└─────────────────────────┘
            │
       ◇ Top dead center? ◇  S204
            │           NO
           YES
            │
┌─────────────────────────┐
│ Horizontally move        │
│ setting unit by set pulse│ ~S205
│ number                   │
└─────────────────────────┘
            │
┌─────────────────────────┐
│ Lower glucose sensor     │
│ by set pulse number      │ ~S206
└─────────────────────────┘
            │
┌─────────────────────────┐
│ Lower sodium ion         │
│ sensor by set pulse      │ ~S207
│ number                   │
└─────────────────────────┘
            │
┌─────────────────────────┐
│ Measure interstitial fluid│
│ collector by glucose     │ ~S208
│ sensor                   │
└─────────────────────────┘
            │
┌─────────────────────────┐
│ Clean sodium ion         │
│ sensor                   │ ~S209
└─────────────────────────┘
            │
           (1)
```

FIG. 25B

FIG. 25C

②

Horizontally move
setting unit — S219

Origin? — S220
NO

↓ YES

Lower sodium ion
sensor by set pulse
number — S221

Clean sodium ion
sensor — S222

Raise sodium ion
sensor — S223

Top dead center? — S224
NO

↓ YES

Return

FIG. 26

```
          ┌─────────────────────┐
          │   Analysis process  │
          └──────────┬──────────┘
                     │  S300
          ┌──────────┴──────────┐
          │ Measure concentration│
          └──────────┬──────────┘
                     │
                  S301
              ╱────────────╲      NO
             ╱ Perspiration ╲────────────┐
             ╲ ratio R       ╱           │
              ╲exceeds      ╱            │
               ╲threshold?╱              │
                ╲────────╱               │
                  │ YES  S303            │  S302
          ┌───────┴──────────┐  ┌────────┴────────────┐
          │Calculate glucose │  │ Create analysis     │
          │      AUC         │  │ result including    │
          └───────┬──────────┘  │ message "Glucose    │
               S304             │ AUC not calculated" │
          ┌───────┴──────────┐  └────────┬────────────┘
          │ Create analysis  │           │
          │ result including │           │
          │ glucose AUC      │           │
          └───────┬──────────┘           │
                  └──────────┬───────────┘
                      ┌──────┴──────┐
                      │   Return    │
                      └─────────────┘
```

EP 3 533 520 A2

FIG. 27

Sodium ion sensor

~22

~222

Glucose sensor

~21

~212

111    110        111    110

~20

FIG. 28

Glucose sensor

21

212

Sodium ion sensor

22

222

110

20

FIG. 29

```
   ┌─────────────────────┐
   ║ Measurement process ║
   └─────────────────────┘
              │
   ┌─────────────────────┐
   │ Horizontally move setting │ ～S400
   │ unit by set pulse number  │
   └─────────────────────┘
              │
   ┌─────────────────────┐
   │ Lower odium ion sensor │ ～S401
   │ by set pulse number    │
   └─────────────────────┘
              │
   ┌─────────────────────┐
   │ Measure interstitial fluid │ ～S402
   │ collector by sodium ion    │
   └─────────────────────┘
              │
   ┌─────────────────────┐
   │ Raise sodium ion sensor │ ～S403
   └─────────────────────┘
              │
          ◇ Top dead center?  S404
            ─── NO
           YES
              │
   ┌─────────────────────┐
   │ Horizontally move setting │ ～S405
   │ unit by set pulse number  │
   └─────────────────────┘
              │
   ┌─────────────────────┐
   │ Move glucose sensor to │ ～S406
   │ measurement position   │
   └─────────────────────┘
              │
   ┌─────────────────────┐
   │ Move sodium ion sensor │ ～S407
   │ to cleaning position   │
   └─────────────────────┘
              │
   ┌─────────────────────┐
   │ Measure interstitial fluid │ ～S408
   │ collector by glucose sensor│
   └─────────────────────┘
              │
   ┌─────────────────────┐
   │ Clean sodium on sensor │ ～S409
   └─────────────────────┘


   ┌─────────────────────┐
   │ Raise glucose and   │ ～S410
   │ sodium ion sensors  │
   └─────────────────────┘
              │
          ◇ Top dead center?  S411
            ─── NO
           YES
              │
   ┌─────────────────────┐
   │ Horizontally move   │ ～S412
   │ setting unit        │
   └─────────────────────┘
              │
          ◇ Origin ?  S413
            ─── NO
           YES
              │
   ┌─────────────────────┐
   │ Lower glucose sensor │ ～S414
   │ by set pulse number  │
   └─────────────────────┘
              │
   ┌─────────────────────┐
   │ Clean glucose sensor │ ～S415
   └─────────────────────┘
              │
   ┌─────────────────────┐
   │ Raise glucose sensor │ ～S416
   └─────────────────────┘
              │
          ◇ Top dead center?  S417
            ─── NO
           YES
              │
         ( Return )
```

FIG. 30

```
┌──────────────────────┐
│‖  Analysis process  ‖│
└──────────────────────┘
            │
┌──────────────────────┐
│ Measure concentration│~ S500
└──────────────────────┘
            │
┌──────────────────────┐
│  Calculate glucose AUC│~ S501
└──────────────────────┘
            │
┌──────────────────────┐
│  Create analysis result│~ S502
│  including glucose AUC │
└──────────────────────┘
            │
     ╭──────────╮
     │  Return  │
     ╰──────────╯
```

FIG. 31

Sodium ion sensor

Glucose sensor

~22

~21

222

212

110

110

~20

FIG. 32

Glucose sensor or
integrated sensor

21

212

20

110

FIG. 33

Measurement process

Horizontally move setting unit by set pulse number — S600

Lower glucose/integrated sensor by set pulse number — S601

Measure interstitial fluid collector by glucose/integrated sensor — S602

Raise glucose/integrated sensor — S603

Top dead center? — S604 — NO

YES

Horizontally move setting unit — S605

Origin? — S606 — NO

YES

Lower glucose/integrated sensor by set pulse number — S607

Clean glucose/integrated sensor — S608

Raise glucose/integrated sensor — S609

Top dead center? — S610 — NO

YES

Return

FIG. 34

```
┌──────────────────────┐
│ ‖ Analysis process ‖  │
└──────────────────────┘
            │
┌──────────────────────┐
│ Measure concentration │  ~S700
└──────────────────────┘
            │
┌──────────────────────┐
│   Calculate glucose   │  ~S701
│         AUC           │
└──────────────────────┘
            │
┌──────────────────────┐
│  Create analysis result │  ~S702
│ including glucose AUC │
└──────────────────────┘
            │
      ╭──────────╮
      │  Return  │
      ╰──────────╯
```

FIG. 35

Glucose sensor or
integrated sensor

21

212

20

110

110

FIG. 36

FIG. 37

Measurement process

Horizontally move setting unit by set pulse number ~S800

Lower integrated sensor and sodium ion sensor by set pulse number ~S801

Measure interstitial fluid collector by integrated sensor and measure perspiration collector by sodium ion sensor ~S802

Raise integrated sensor and sodium ion sensor ~S803

S804 Top dead center? — NO

YES

Horizontally move setting unit ~S805

S806 Origin? — NO

YES

Lower integrated sensor and sodium ion sensor by set pulse number ~S807

Clean integrated sensor and sodium ion sensor ~S808

Raise integrated sensor and sodium ion sensor ~S809

S810 Top dead center? — NO

YES

Return

FIG. 38

FIG. 39

FIG. 40

**REFERENCES CITED IN THE DESCRIPTION**

**Patent documents cited in the description**

- WO 2010013808 A

- JP 2012217667 A